# EUROPÄISCHE PATENTSCHRIFT

(11) **EP 3 886 804 B1**
(45) Veröffentlichungstag und Bekanntmachung des Hinweises auf die Patenterteilung: **02.07.2025**
(21) Anmeldenummer: 19795509.9
(22) Anmeldetag: 23.10.2019
(51) Int. Cl.: A61K 9/00, A61K 9/08, A61K 47/10, A61P 31/10, A61K 47/26, A61K 47/32, A61K 47/34, A61K 31/53, A61P 17/02, A61P 43/00

(54) **WÄSSRIGE ZUSAMMENSETZUNG, INSBESONDERE ZUR BEHANDLUNG VON SCHLEIMHAUT UND/ODER WUNDEN**
AQUEOUS COMPOSITION, IN PARTICULAR FOR TREATING MUCOSA AND/OR WOUNDS
COMPOSITION AQUEUSE, EN PARTICULIER POUR TRAITER UNE MUQUEUSE ET/OU DES PLAIES

(30) Priorität: 29.11.2018 DE 102018220624
(43) Veröffentlichungstag der Anmeldung: 06.10.2021
(73) Patentinhaber: B. Braun Melsungen AG, 34212 Melsungen (DE)
(72) Erfinder: ARNDT, Andreas, 6010 Kriens (CH); DÜCK, Natalie, 6207 Nottwil (CH); KURZ, Michael, 6208 Oberkirch (CH)
(74) Vertreter: Patentanwälte Ruff, Wilhelm, Beier, Dauster & Partner mbB
(86) Internationale Anmeldenummer: PCT/EP2019/078868
(87) Internationale Veröffentlichungsnummer: WO 2020/108880

(56) Entgegenhaltungen:
- EP-A1- 1 574 503
- THOMPSON P E ET AL: "STUDIES ON A DIHYDROTRIAZINE AND A SULFONE, ALONE AND IN COMBINATION, AGAINST PLASMODIUM BERGHEI IN MICE", AMERICAN JOURNAL OF TROPICAL MEDICINE & HYGIENE, AMERICAN SOCIETY OF TROPICAL MEDICINE AND HYGIENE, US, vol. 14, 1 March 1965 (1965-03-01), pages 198 - 206, XP009161567, ISSN: 0002-9637

## Beschreibung

### ANWENDUNGSGEBIET UND STAND DER TECHNIK

Die Erfindung betrifft eine wässrige Zusammensetzung, welche sich insbesondere zur Behandlung von Schleimhaut und/oder Wunden eignet.

Für die Behandlung von Schleimhaut und Wunden haben sich iodhaltige Produkte etabliert, welche das lod in Form von lodophoren, z.B. Povidon-lod, enthalten. Diese zeichnen sich durch eine gute Verträglichkeit aus. Auf Grund möglicher Allergien und Sensibilisierungen aber auch der bekannten Resorption von lod durch den Organismus ist der Einsatz dieser Mittel limitiert.

Aus diesem Grund wurden Produkte mit kationischen antimikrobiellen Wirkstoffen vorgeschlagen.

Eine wässrige, Polyhexamethylenbiguanid enthaltende Zusammensetzung, welche insbesondere in Form einer Wundspüllösung oder eines Wundgels vorliegen kann, ist aus der WO 03/004013 A1 bekannt.

Gegenstand der DE 102 05 883 A1 ist ein wässriges Antiseptikum auf Basis von Bispyridiniumalkanen.

Die WO 2007/031520 A2 betrifft die Verwendung von Octenidindihydrochlorid zur Herstellung einer pharmazeutischen Zusammensetzung unter anderem zur Behandlung von Wunden.

Die EP 1 574 503 A1 offenbart antibakteriell wirksame Dihydrotriazinverbindungen.

Thompson P. E. et. al.: ("STUDIES ON A DIHYDROTRIAZINE AND A SULFONE, ALONE AND IN COMBINATION, AGAINST PLASMODIUM BERGHEl IN MICE", American Journal of Tropical Medicine & Hygiene, Bd. 14, 1. März 1965, S. 198-206) offenbart Dihydrotriazinverbindungen zur Behandlung von Malaria.

Konventionelle Mittel haben den Nachteil, dass sie Wirklücken gegen Pilze, insbesondere Hefen, aufweisen.

Unter dem Namen Octenisept ist ein octenidinhaltiges Präparat erhältllich, welches zwar eine gute Schleimhautverträglichkeit vorweisen kann, bei der Anwendung in tieferen Wunden jedoch zu Unverträglichkeiten bis hin zu Nekrosen führen kann. Dies wurde unter anderem beim deutschen Bundesinstitut für Arzneimittel und Medizinprodukte veröffentlicht: (https://www.bfarm.de/SharedDocs/Risikoinformationen/Pharmakovigilanz/DE/RHB/201 1/rhboctenisept.html).

Eine octenidinhaltige Zusammensetzung ist aus der EP 0 411 315 A1 bekannt.

### AUFGABE UND LÖSUNG

Der Erfindung liegt die Aufgabe zugrunde, eine sehr gut verträgliche Zusammensetzung bereitzustellen, welche sich zum Behandeln von Schleimhaut und/oder Wunden eignet und gleichzeitig eine gegenüber Pilzen, insbesondere Hefen, verbesserte Wirksamkeit aufweist.

Diese Aufgabe wird erfindungsgemäß gelöst durch eine wässrige Zusammensetzung mit den Merkmalen des unabhängigen Anspruchs 1. Bevorzugte Ausgestaltungen der wässrigen Zusammensetzung sind Gegenstand der abhängigen Ansprüche sowie der Beschreibung. Der Wortlaut sämtlicher Ansprüche wird hiermit durch ausdrückliche Bezugnahme zum Inhalt der Beschreibung gemacht.

Bei der erfindungsgemäßen Zusammensetzung handelt es sich um eine wässrige, d.h. Wasser enthaltende, Zusammensetzung. Vorzugsweise liegt die wässrige Zusammensetzung in Form einer wässrigen Lösung, d.h. in Form einer Wasser enthaltenden Lösung, oder in Form eines Hydrogels, d.h. in Form eines Wasser enthaltenden Gels, vor.

Die wässrige Zusammensetzung weist Folgendes auf:
- eine Dihydrotriazinverbindung der nachstehenden allgemeinen Formel I: 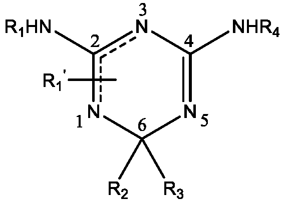 wobei
   - R₁ (i) eine Phenylgruppe oder eine Phenylalkylgruppe, von denen jede optional mit 1 bis 3 Substituenten, ausgewählt aus der Gruppe bestehend aus C₁₋₆-Alkoxygruppe, Hydroxygruppe, einem Halogenatom, C₁₋₆-Halogenalkylgruppe, C₁₋₆-Alkylgruppe, einer Sulfonamidogruppe und C₁₋₆-Halogenalkoxygruppe, substituiert ist, (ii) eine Naphthylgruppe oder eine Naphthylalkylgruppe, (iii) eine heterocyclische Gruppe, eine heterocyclische Alkylgruppe oder eine heterocyclische Aminoalkylgruppe, (iv) eine Alkylgruppe mit 1 bis 16 Kohlenstoffatomen oder (v) eine Cycloalkylgruppe oder eine Cycloalkyl-alkylgruppe bedeutet,
   - R₁' ein Wasserstoffatom, das an Position 1 oder 3 des Dihydrotriazinrings an das Stickstoffatom gebunden ist, bedeutet,
   - R₂ und R₃ unabhängig voneinander ein Wasserstoffatom oder eine Methylgruppe bedeuten,
   - R₄ eine Alkylgruppe mit 7 bis 16 Kohlenstoffatomen bedeutet und
   - die Strichlinie angibt, dass die Stellung einer Doppelbindung entweder zwischen den Positionen 1 und 2 oder zwischen den Positionen 2 und 3 des Dihydrotriazinrings liegt,
   oder ein Tautomer davon, d.h. ein Tautomer der Dihydrotriazinverbindung der allgemeinen Formel I, oder ein Salz, insbesondere pharmakologisch akzeptables Salz, davon, d.h. ein Salz, insbesondere pharmakologisch akzeptables Salz, der Dihydrotriazinverbindung der allgemeinen Formel I, und
- einen Entschäumer
zur Anwendung bei der Vorbeugung oder Behandlung von durch Pilze verursachte oder mitverursachte Infektionen und/oder Infektionskrankheiten.

Die wässrige Zusammensetzung eignet sich insbesondere zur Vorbeugung, d.h. Prophylaxe, oder Behandlung von Schleimhaut und/oder Wunden, bevorzugt akuten oder chronischen Wunden, und/oder zur Vorbeugung, d.h. Prophylaxe, oder Behandlung von Infektionen und/oder zur Vorbeugung, d.h. Prophylaxe, oder Behandlung von Infektionskrankheiten, wie beispielsweise Wundrose oder Erysipel. Die wässrige Zusammensetzung kann daher im Sinne der vorliegenden Erfindung auch als wässrige Wundbehandlungszusammensetzung, insbesondere wässrige antiseptische Zusammensetzung, bezeichnet werden. Bevorzugt handelt es sich bei der wässrigen Zusammensetzung um eine wässrige Wundspüllösung.

Unter dem Ausdruck "Dihydrotriazinverbindung" soll im Sinne der vorliegenden Erfindung eine Verbindung mit einem sechsgliedrigen, lediglich zwei Doppelbindungen aufweisenden Triazinring, d.h. einem sogenannten Dihydrotriazinring, verstanden werden, wobei der Triazinring bzw. Dihydrotriazinring an den Positionen 1, 3 und 5 jeweils ein Ringstickstoffatom und an den Positionen 2, 4, und 6 jeweils ein Ringkohlenstoffatom aufweist und wobei die Stellung der einen Doppelbindung zwischen den Positionen 4 und 5 des Triazinings bzw. Dihydrotriazinrings und die Stellung der anderen, d.h. zweiten oder verbleibenden, Doppelbindung entweder zwischen den Positionen 1 und 2 oder zwischen den Positionen 2 und 3 des Triazinrings bzw. Dihydrotriazinrings liegt.

Unter dem Ausdruck "Entschäumer" soll im Sinne der vorliegenden Erfindung eine Verbindung verstanden werden, welche in der Lage ist, eine Schaumbildung zu verlangsamen abzuschwächen, d.h. abzumildern oder zu reduzieren, oder zu vermeiden.

Unter dem Ausdruck "Tensid" soll im Sinne der vorliegenden Erfindung eine Verbindung verstanden werden, welche die Oberflächenspannung einer Flüssigkeit, insbesondere von Wasser oder einer wässrigen Flüssigkeit, und/oder die Grenzflächenspannung zwischen zwei Phasen herabsetzt und die Bildung von Dispersionen ermöglicht oder unterstützt und/oder als Lösungsvermittler wirkt.

Unter dem Ausdruck "Emulgator" soll im Sinne der vorliegenden Erfindung eine Verbindung verstanden werden, welche in der Lage ist, zwei nicht miteinander mischbare Flüssigkeiten, wie beispielsweise Öl und Wasser, zu einer Emulsion zu vermengen und insbesondere zu stabilisieren.

Der Ausdruck "Pilze" kann im Sinne der vorliegenden Erfindung einzellige oder vielzellige Pilze bedeuten.

Unter dem Ausdruck "Hefen" sollen im Sinne der vorliegenden Erfindung einzellige Pilze verstanden werden, welche sich durch Sprossung oder Teilung (Spaltung) vermehren (sogenannte Hefepilze).

Unter dem Ausdruck "Phenlygruppe" soll im Sinne der vorliegenden Erfindung ein Benzolrest, also die Atomgruppe -C₆H₅, verstanden werden.

Unter dem Ausdruck "Benzlygruppe" soll im Sinne der vorliegenden Erfindung die Phenylmethylgruppe -CH₂-C₆H₅, früher auch als α-Tolylgruppe bezeichnet, verstanden werden.

Der Ausdruck "Phenylalkylgruppe" bedeutet im Sinne der vorliegenden Erfindung eine Gruppe, bei welcher eine lineare, d.h. unverzweigte, oder verzweigte Alkylgruppe oder Alkylengruppe, insbesondere mit 1 bis 6 Kohlenstoffatomen, an eine Phenylgruppe gebunden ist. Die Alkylgruppe oder Alkylengruppe kann dabei eine substituierte oder unsubstituierte Alkylgruppe oder Alkylengruppe sein. Bevorzugt handelt es sich bei der Phenylalkylgruppe um eine Benzylgruppe, Methylbenzlygruppe wie insbesondere 4-Methylbenzylgruppe, 1-Phenylethylgruppe, 2-Phenylethylgruppe, 1-Phenylpropylgruppe, 2-Phenylpropylgruppe oder 3-Phenylpropylgruppe.

Ein Benzolring der Phenylgruppe oder Phenylalkylgruppe kann ein bis drei Substituenten aufweisen, insbesondere ausgewählt aus der Gruppe bestehend aus Halogenatom, Hydroxygruppe, C₁₋₆-Alkylgruppe, C₁₋₆-Haloalkylgruppe, C₁₋₆-Alkoxygruppe, C₁₋₆-Haloalkoxygruppe und Sulfonamidogruppe.

Der Ausdruck "Halogenatom" kann im Sinne der vorliegenden Erfindung ein Fluoratom, ein Chloratom, ein Bromatom oder ein lodatom bedeuten.

Der Ausdruck "C₁₋₆-Alkylgruppe" kann im Sinne der vorliegenden Erfindung eine entsprechende, d.h. eine 1 bis 6 Kohlenstoffatome aufweisende, lineare oder verzweigte Alkylgruppe bedeuten. Die Alkylgruppe kann dabei eine substituierte oder unsubstituierte Alkylgruppe sein. Beispielsweise kann es sich bei der C₁₋₆-Alkylgruppe um eine Methylgruppe, Ethylgruppe, n-Propylgruppe, Isopropylgruppe, n-Butylgruppe, Isobutylgruppe, sec-Butylgruppe, tert-Butylgruppe, n-Pentylgruppe, sec-Pentylgruppe, Isopentylgruppe, Neopentylgruppe, n-Hexylgruppe oder Isohexylgruppe handeln.

Der Ausdruck "C₁₋₆-Haloalkylgruppe" kann im Sinne der vorliegenden Erfindung insbesondere eine Chloromethylgruppe, Bromomethylgruppe, 1-Chloroethylgruppe oder Trifluoromethylgruppe bedeuten.

Der Ausdruck "C₁₋₆-Alkoxygruppe" kann im Sinne der vorliegenden Erfindung insbesondere eine Methoxygruppe, Ethoxygruppe, n-Propoxygruppe, Isopropoxygruppe, n-Butoxygruppe oder Isobutoxygruppe bedeuten.

Der Ausdruck "C₁₋₆-Haloalkoxygruppe" kann im Sinne der vorliegenden Erfindung insbesondere eine Trifluoromethoxygruppe bedeuten.

Der Ausdruck "Naphthylgruppe" kann im Sinne der vorliegenden Erfindung eine 1-Naphthylgruppe oder eine 2-Naphthylgruppe bedeuten.

Der Ausdruck "Naphthylalkylgruppe" bedeutet im Sinne der vorliegenden Erfindung eine Gruppe, bei welcher eine lineare, d.h. unverzweigte, oder verzweigte Alkylgruppe, insbesondere mit 1 bis 6 Kohlenstoffatomen, an eine Naphthylgruppe gebunden ist. Die Alkylgruppe kann dabei eine substituierte oder unsubstituierte Alkylgruppe sein. Bevorzugt handelt es sich bei der Naphthylalkylgruppe um eine 1-Naphthylmethylgruppe, 2-Naphthylmethylgruppe, 1-Naphthylethylgruppe oder 2-Naphthylethylgruppe.

Der Ausdruck "heterocyclische Gruppe" bedeutet im Sinne der vorliegenden Erfindung eine drei- bis sechsgliedrige heterocyclische Gruppe, welche ein bis drei Atome, ausgewählt aus der Gruppe bestehend aus Stickstoffatom, Sauerstoffatom und Schwefelatom, enthält, wobei an die heterocyclische Gruppe ein Benzolring anelliert oder kondensiert sein kann. Die heterocyclische Gruppe kann beispielsweise eine Pyridylgruppe, eine Pyrazinylgruppe, eine Thiazolylgruppe, eine Piperidylgruppe, eine Piperazylgruppe, eine Tetrahydrofurylgruppe, eine Thienylgruppe, eine Pyrrolylgruppe, eine Pyrrolidinylgruppe, eine Oxazolylgruppe, eine Imidazolylgruppe, eine Isooxazolylgruppe, eine Isothiazolylgruppe, eine Pyrazolylgruppe, eine Tetrahydropyranylgruppe, eine 2-Oxotetrahydropyranylgruppe, eine Pyrimidinylgruppe, eine Pyradizinylgruppe, eine Morpholinylgruppe, eine 1,3,5-Triazinylgruppe, eine 1,2,4-Triazinylgruppe, eine Quinolylgruppe oder eine Isoquinolylgruppe bedeuten.

Insbesondere kann die heterocyclische Gruppe im Sinne der vorliegenden Erfindung eine 2-Pyridylgruppe, eine 3-Pyridylgruppe, eine 4-Pyridylgruppe, eine 2-Furylgruppe, eine 2-Thiazolylgruppe, eine 1-Piperidylgruppe, eine 1-Piperazylgruppe, eine 2-Quinolylgruppe, eine 3-Quinolylgruppe, eine 4-Quinolylgruppe, eine 5-Quinolylgruppe, eine 8-Quinolylgruppe, eine 1-Isoquinolylgruppe, eine 3-Isoquinolylgruppe, eine 4-Isoquinolylgruppe oder eine 5-Isoquinolylgruppe bedeuten.

Der Ausdruck "heterocyclische Alkylgruppe" bedeutet im Sinne der vorliegenden Erfindung eine Gruppe, bei welcher eine lineare, d.h. unverzweigte, oder verzweigte Alkylgruppe, insbesondere mit 1 bis 6 Kohlenstoffatomen, an eine heterocyclische Gruppe, insbesondere wie in den vorherigen Absätzen definiert oder beschrieben, gebunden ist. Die Alkylgruppe kann dabei eine substituierte oder unsubstituierte Alkylgruppe sein. Bevorzugt handelt es sich bei der heterocyclischen Alkylgruppe um eine 2-Pyridylmethylgruppe, 3-Pyridylmethylgruppe, 4-Pyridylmethylgruppe, 2-Pyridylethylgruppe, 3-Pyridylethylgruppe, 4-Pyridylethylgruppe, Pyrazinylmethylgruppe, Pyrazinylethylgruppe, 2-Furylmethylgruppe, 2-Furylethylgruppe, 2-Thiazolylmethylgruppe, 2-Thiazolylethylgruppe, 4-Piperidylmethylgruppe, 2-Quinolylmethylgruppe, 3-Quinolylmethylgruppe, 4-Quinolylmethylgruppe, 5-Quinolylmethylgruppe, 8-Quinolylmethylgruppe, 1-Isoquinolylmethylgruppe, 3-Isoquinolylmethylgruppe, 4-Isoquinolylmethylgruppe oder 5-Isoquinolylmethylgruppe.

Der Ausdruck "heterocyclische Aminoalkylgruppe" bedeutet im Sinne der vorliegenden Erfindung eine Gruppe, bei welcher eine lineare, d.h. unverzweigte, oder verzweigte Alkylgruppe, insbesondere mit 1 bis 12 Kohlenstoffatomen, an eine heterocyclische Aminogruppe gebunden ist. Die Alkylgruppe kann dabei eine substituierte oder unsubstituierte Alkylgruppe sein. Bevorzugt handelt es sich bei der heterocyclischen Aminoalkylgruppe um eine 4-Amino-dihydro-1,3,5-triazin-2-yl-aminogruppe, eine 4-Alkylamino-dihydro-1,3,5-triazin-2-yl-aminogruppe oder 4-Phenylalkylamino-dihydro-1,3,5-triazin-2-yl-aminogruppe.

Der Ausdruck "Alkylgruppe mit 1 bis 16 Kohlenstoffatomen" bedeutet im Sinne der vorliegenden Erfindung eine lineare, d.h. unverzweigte, oder verzweigte Alkylgruppe mit 1 bis 16 Kohlenstoffatomen. Die Alkylgruppe kann dabei eine substituierte oder unsubstituierte Alkylgruppe sein. Bevorzugt handelt es sich bei der Alkylgruppe mit 1 bis 16 Kohlenstoffatomen um eine Methylgruppe, Ethylgruppe, n-Propylgruppe, Isopropylgruppe, n-Butylgruppe, Isobutylgruppe, sec-Butylgruppe, tert-Butylgruppe, n-Hexylgruppe, n-Heptylgruppe, n-Octylgruppe, tert-Octylgruppe, n-Nonylgruppe, n-Decylgruppe, n-Undecylgruppe, n-Dodecylgruppe, n-Tridecylgruppe, n-Tetradecylgruppe, n-Pentadecylgruppe oder n-Hexadecylgruppe.

Der Ausdruck "Cycloalkylgruppe" bedeutet im Sinne der vorliegenden Erfindung insbesondere eine Cycloalkylgruppe mit 3 bis 6 Kohlenstoffatomen. Beispielsweise kann es sich bei der Cycloalkylgruppe um eine Cyclopropylgruppe, Cyclobutylgruppe, Cyclopentylgruppe oder Cyclohexylgruppe handeln.

Der Ausdruck "Cycloalkyl-alkylgruppe" bedeutet im Sinne der vorliegenden Erfindung insbesondere eine Gruppe, bei welcher eine lineare, d.h. unverzweigte, oder verzweigte Alkylgruppe, insbesondere mit 1 bis 6 Kohlenstoffatomen, an eine Cycloalkylgruppe, insbesondere wie im vorherigen Absatz definiert oder beschrieben, gebunden ist. Die Alkylgruppe kann dabei eine substituierte oder unsubstituierte Alkylgruppe sein. Bevorzugt handelt es sich bei der Cycloalkyl-alkylgruppe um eine Cyclohexylmethylgruppe, eine 1-Cyclohexylethylgruppe oder eine 2-Cyclohexylethylgruppe.

Der Ausdruck "Alkylgruppe mit 7 bis 16 Kohlenstoffatomen" bedeutet im Sinne der vorliegenden Erfindung eine lineare, d.h. unverzweigte, oder verzweigte Alkylgruppe mit 7 bis 16 Kohlenstoffatomen. Die Alkylgruppe kann dabei eine substituierte oder unsubstituierte Alkylgruppe sein. Bevorzugt handelt es sich bei der Alkylgruppe mit 7 bis 16 Kohlenstoffatomen um eine n-Heptylgruppe, n-Octylgruppe, tert-Octylgruppe, n-Nonylgruppe, n-Decylgruppe, n-Undecylgruppe, n-Dodecylgruppe, n-Tridecylgruppe, n-Tetradecylgruppe, n-Pentadecylgruppe oder n-Hexadecylgruppe.

Der im Zusammenhang der Dihydrotriazinverbindung der allgemeinen Formel I verwendete Ausdruck "Salz" kann im Sinne der vorliegenden Erfindung insbesondere ein Salz mit einer organischen Säure, wie beispielsweise Ameisensäure, Essigsäure, Propionsäure, Milchsäure, Buttersäure, Isobuttersäure, Äpfelsäure, Maleinsäure, Malonsäure, Fumarsäure, Bernsteinsäure, Bernsteinsäuremonoamid, Glutarsäure, Weinsäure, Oxalsäure, Citronensäure, Glykolsäure, Glucuronsäure, Ascorbinsäure, Asparaginsäure, Glutaminsäure, Benzoesäure, Phthalsäure, Salicylsäure, Anthranilsäure, Benzolsulfonsäure, p-Toluolsulfonsäure oder Methansulfonsäure, bedeuten.

Unter dem Ausdruck "antiseptische Zusammensetzung" soll im Sinne der vorliegenden Erfindung eine Zusammensetzung zur Reduktion von Mikroorganismen auf belebten Oberflächen, insbesondere auf Schleimhaut und/oder Wunden, und/oder zur Vorbeugung, d.h. Prophylaxe, oder Behandlung von Schleimhaut und/oder Wunden und/oder zur Vorbeugung und/oder Behandlung von Infektionen und/oder Infektionskrankheiten verstanden werden.

Die vorliegende Erfindung beruht zum Einen auf der überraschenden Erkenntnis, dass Dihydrotriazinverbindungen der allgemeinen Formel I oder ein Tautomer davon oder ein Salz davon eine gegenüber gattungsgemäßen Antiseptika, wie beispielsweise Polyhexamethylenbiguanid oder Octenidinhydrochlorid, verbesserte Wirksamkeit gegenüber Pilzen, insbesondere Hefen, aufweisen. Dadurch ist in besonders vorteilhafter Weise eine bessere Vorbeugung oder Behandlung von insbesondere durch Pilze verursachten oder mitverursachten Wunden und/oder Infektionen und/oder Infektionskrankheiten möglich.

Weiterhin stellte sich überraschenderweise heraus, dass durch Kombination einer Dihydrotriazinverbindung der allgemeinen Formel I oder eines Tautomers davon oder eines Salzes davon und eines Entschäumers eine schaumarme oder schaumfreie Zusammensetzung erhältlich ist, wodurch eine Verwendung der Dihydrotriazinverbindung insbesondere zur Vorbeugung oder Behandlung von Schleimhaut und/oder Wunden und/oder Infektionen und/oder Infektionskrankheiten optimiert oder überhaupt erst ermöglicht wird. Eine Verwendung von Dihydrotriazinverbindungen der allgemeinen Formel I oder von Tautomeren davon oder von Salzen davon zur Behandlung und/oder Spülung von Schleimhaut und/oder Wunden war aufgrund von deren (zum Teil) extremen Schaumverhalten nicht zu erwarten.

### In Ausgestaltung der Erfindung bedeutet/bedeuten

- R₁ eine Phenylgruppe oder eine Phenylalkylgruppe von denen jede optional durch 1 bis 3 Substituenten, ausgewählt aus der Gruppe bestehend aus Fluoratom, Chloratom, Hydroxygruppe, Methylgruppe, tert-Butylgruppe, Trifluoromethylgruppe und Methoxygruppe, substituiert ist,
- R₁' ein Wasserstoffatom, das an Position 1 oder 3 des Dihydrotriazinrings an das Stickstoffatom gebunden ist,
- R₂ und R₃ jeweils eine Methylgruppe und
- R₄ eine n-Octylgruppe, n-Nonylgruppe oder n-Decylgruppe.

### Bevorzugt bedeutet/bedeuten

- R₁ eine Phenylgruppe, eine Benzylgruppe oder eine 2-Phenylethylgruppe, von denen jede optional durch 1 bis 3 Substituenten, ausgewählt aus der Gruppe bestehend aus Fluoratom, Chloratom, Hydroxygruppe, Methylgruppe, tert-Butylgruppe, Trifluoromethylgruppe und Methoxygruppe, substituiert ist,
- R₁' ein Wasserstoffatom, das an Position 1 oder 3 des Dihydrotriazinrings an das Stickstoffatom gebunden ist,
- R₂ und R₃ jeweils eine Methylgruppe und
- R₄ eine n-Octylgruppe, n-Nonylgruppe oder n-Decylgruppe.

### Besonders bevorzugt bedeutet/bedeuten

- R₁ eine Benzylgruppe, welche optional durch 1 bis 3 Substituenten, ausgewählt aus der Gruppe bestehend aus Fluoratom, Chloratom, Hydroxygruppe, Methylgruppe, tert-Butylgruppe, Trifluoromethylgruppe und Methoxygruppe, bevorzugt durch 1 bis 3 Methylgruppen, besonders bevorzugt durch eine Methylgruppe, substituiert ist,
- R₁' ein Wasserstoffatom, das an Position 1 oder 3 des Dihydrotriazinrings an das Stickstoffatom gebunden ist,
- R₂ und R₃ jeweils eine Methylgruppe und
- R₄ eine n-Octylgruppe, n-Nonylgruppe oder n-Decylgruppe.

### In weiterer Ausgestaltung der Erfindung bedeutet/bedeuten

- R₁ eine Phenylgruppe, 4-Chlorophenylgruppe, 2,4-Difluorophenylgruppe, 2,3,4-Trifluorophenylgruppe, 4-tert-Butylphenylgruppe, 4-Methoxyphenylgruppe, 2-Methoxy-4-tert-butylphenylgruppe, 4-Trifluoromethoxyphenylgruppe, Benzylgruppe, Methylbenzlygruppe wie insbesondere 4-Methylbenzylgruppe, 4-Methoxybenzylgruppe, 3,4-Dimethoxybenzylgruppe, 4-Hydroxybenzylgruppe, 3,4-Dichlorobenzylgruppe, 2,3,4-Trichlorobenzylgruppe, 4-Trifluoromethylbenzylgruppe, 1-Phenylethylgruppe, 2-Phenylethylgruppe, 1-Phenylpropylgruppe, 2-Phenylpropylgruppe oder 3-Phenylpropylgruppe, bevorzugt eine Methylbenzylgruppe, besonders bevorzugt eine 4-Methylbenzylgruppe,
- R₁' ein Wasserstoffatom, das an Position 1 oder 3 des Dihydrotriazinrings an das Stickstoffatom gebunden ist,
- R₂ und R₃ jeweils eine Methylgruppe und
- R₄ eine n-Octylgruppe, n-Nonylgruppe oder n-Decylgruppe.

In weiterer Ausgestaltung der Erfindung bedeutet/bedeuten R₁ eine Methylbenzylgruppe, vorzugsweise 4-Methylbenzylgruppe, und/oder R₂ und R₃ jeweils eine Methylgruppe und/oder R₄ eine n-Octylgruppe. Bevorzugt bedeutet/bedeuten R₁ eine Methylbenzylgruppe, vorzugsweise 4-Methylbenzylgruppe, R₂ und R₃ jeweils eine Methylgruppe und R₄ eine n-Octylgruppe.

In weiterer Ausgestaltung der Erfindung bedeutet/bedeuten R₁ eine Methylbenzylgruppe, vorzugsweise 4-Methylbenzylgruppe, R₁' ein Wasserstoffatom, das an Position 1 oder 3 des Dihydrotriazinrings an das Stickstoffatom gebunden ist, R₂ und R₃ jeweils eine Methylgruppe und R₄ eine n-Octylgruppe

Besonders bevorzugt weist die Dihydrotriazinverbindung die nachfolgende Formel la auf:

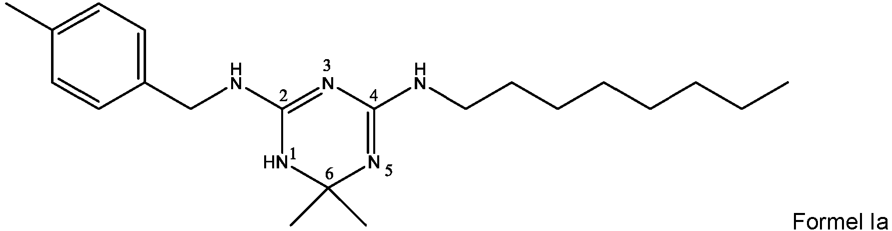

Alternativ kann die Dihydrotriazinverbindung die nachfolgende Formel Ib aufweisen:

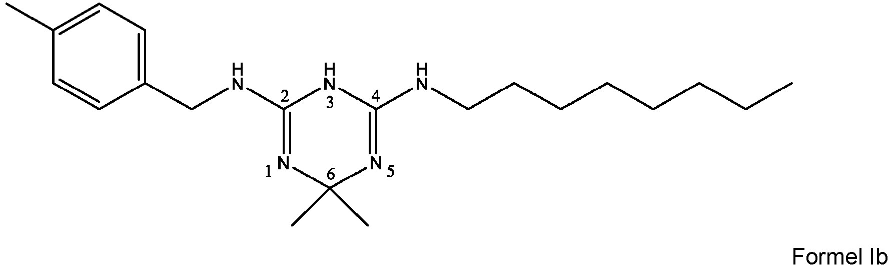

In weiterer Ausgestaltung der Erfindung handelt es sich bei der Dihydrotriazinverbindung oder dem Salz davon um 4-Octylamino-1,6-dihydro-6,6-dimethyl-2-(4'-methylbenzylamino)-1,3,5-triazingluconat, welches auch als 6,6-Dimethyl-N²-(4-methylbenzyl)-N⁴-octyl-1,6-dihydro-[1,3,5]triazin-2,4-diamin-gluconat bezeichnet werden kann, oder um ein Tautomer davon. Vorzugsweise handelt es sich bei der Dihydrotriazinverbindung oder dem Salz davon um 4-Octylamino-1,6-dihydro-6,6-dimethyl-2-(4'-methylbenzylamino)-1,3,5-triazin-D-gluconat, welches auch als 6,6-Dimethyl-N²-(4-methylbenzyl)-N⁴-octyl-1,6-dihydro-[1,3,5]triazin-2,4-diamin-D-gluconat bezeichnet werden kann, oder um ein Tautomer davon. Die in diesem Absatz offenbarte Dihydrotriazinverbindung bzw. das in diesem Absatz offenbarte Dihydrotriazinverbindungssalz hat sich als besonders wirksam zur Vorbeugung oder Behandlung von Schleimhaut und/oder Wunden und/oder Infektionen und/oder Infektionskrankheiten, insbesondere von durch Pilze, insbesondere Hefen, verursachte oder mitverursachte Wunden und/oder Infektionen und/oder Infektionskrankheiten, herausgestellt.

Die im vorherigen Absatz erwähnte Dihydrotriazinverbindung bzw. das im vorherigen Absatz erwähnte Dihydrotriazinverbindungssalz 4-Octylamino-1,6-dihydro-6,6-dimethyl-2-(4'-methylbenzylamino)-1,3,5-triazin-D-gluconat ist unter der Bezeichnung "Femotaxidine" kommerziell erhältlich und kann durch nachfolgende Formel Ia^{#} dargestellt werden:

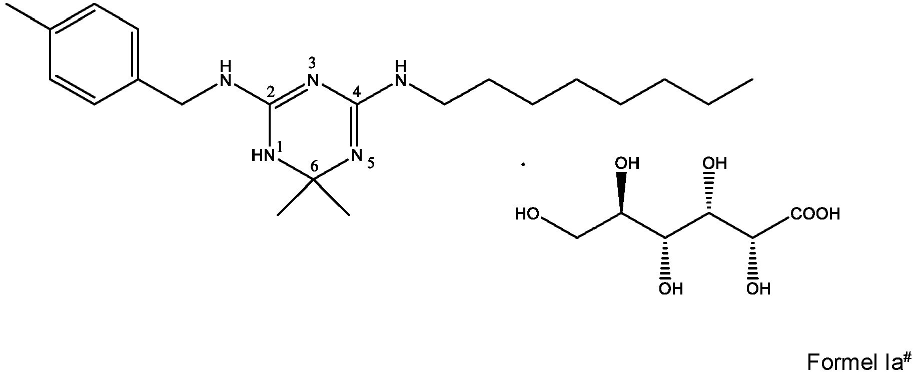

In weiterer Ausgestaltung der Erfindung handelt es sich bei der Dihydrotriazinverbindung der allgemeinen Formel I oder dem Salz davon um 4-Octylamino-3,6-dihydro-6,6-dimethyl-2-(4'-methylbenzylamino)-1,3,5-triazingluconat, insbesondere 4-Octylamino-3,6-dihydro-6,6-dimethyl-2-(4'-methylbenzylamino)-1,3,5-triazin-D-gluconat, oder ein Tautomer davon.

In weiterer Ausgestaltung der Erfindung weist die Dihydrotriazinverbindung der allgemeinen Formel I oder das Tautomer davon oder das Salz davon einen Anteil von 0.001 Gew.-% bis 1.00 Gew.-%, insbesondere 0.01 Gew.-% bis 0.50 Gew.-%, vorzugsweise 0.025 Gew.-% bis 0.25 Gew.-%, auf, bezogen auf das Gesamtgewicht der wässrigen Zusammensetzung. Insbesondere die in diesem Absatz offenbarten Anteile für die Dihydrotriazinverbindung oder das Tautomer davon oder das Salz davon haben sich als besonders vorteilhaft im Hinblick auf die Vorbeugung oder Behandlung von Schleimhaut und/oder Wunden und/oder Infektionen und/oder Infektionskrankheiten, insbesondere von durch Pilze, insbesondere Hefen, verursachte oder mitverursachte Wunden und/oder Infektionen und/oder Infektionskrankheiten, herausgestellt.

In weiterer Ausgestaltung der Erfindung ist der Entschäumer ausgewählt aus der Gruppe bestehend aus Alkylamid, Silikon, Poloxamer und Kombinationen, insbesondere Mischungen, von wenigstens zwei der genannten Entschäumer.

Das Alkylamid kann insbesondere ein Alkylamid der Formel R₁-NH-R₂ sein, wobei es sich bei R₁ um n-Octyl, iso-Octyl oder 2-Ethylhexyl und bei R₂ um n- oder iso-Octan, n- oder iso-Nonan oder n- oder iso-Decan handelt. Erfindungsgemäß kann es sich bei dem Alkylamid auch um eine Kombination, insbesondere Mischung, von wenigstens zwei entsprechender Alkylamide handeln.

Bei dem Silikon kann es sich um ein Polydimethylsiloxan, Polyether-Siloxan (Siloxan-Polyethylenglykol oder Siloxan-Polypropylenglykol), 3D-modifiziertes Siloxan, auch Crosslink-Siloxan genannt, oder eine Kombination, insbesondere Mischung, von wenigstens zwei der genannten Silikone handeln.

Bevorzugt weist das Poloxamer 2 bis 130 Struktureinheiten -CH₂-CH₂-O- und/oder 15 bis 67 Struktureinheiten -CHCH₃-CH₂-O- pro Molekül auf.

Unter dem Ausdruck "Poloxamer" soll im Sinne der vorliegenden Erfindung ein Blockcopolymer aus Ethylenoxid und Propylenoxid verstanden werden.

Insbesondere kann es sich bei dem Poloxamer um Poloxamer 407, Poloxamer 188 oder um eine Kombination, insbesondere Mischung, davon handeln.

Weiterhin kann es sich bei dem Entschäumer um eine Kombination, insbesondere Mischung, von einem Alkylamid und einem Silikon handeln. Eine solche Kombination von Entschäumern ist besonders vorteilhaft im Hinblick auf die Unterdrückung oder Abschwächung einer Schaumbildung der Dihydrazinverbindung oder des Tautomers davon oder des Salzes davon. In diesem Fall kann das Alkylamid einen Anteil, insbesondere Aktiv-Anteil, von 0.0001 Gew.-% bis 0.1 Gew.-%, insbesondere 0.0005 Gew.-% bis 0.05 Gew.-%, vorzugsweise 0.001 Gew.-% bis 0.05 Gew.-%, bezogen auf das Gesamtgewicht der wässrigen Zusammensetzung, und das Silikon einen Anteil, insbesondere Aktiv-Anteil, von 0.00001 Gew.-% bis 0.01 Gew.-%, insbesondere 0.00002 Gew.-% bis 0.005 Gew.-%, vorzugsweise 0.00005 Gew.-% bis 0.005 Gew.-%, bezogen auf das Gesamtgewicht der wässrigen Zusammensetzung, aufweisen.

In weiterer Ausgestaltung der Erfindung weist der Entschäumer einen Anteil von 0.0001 Gew.-% bis 2.0 Gew.-%, insbesondere 0.0005 Gew.-% bis 1.5 Gew.-%, vorzugsweise 0.001 Gew.-% bis 1.0 Gew.-%, auf, bezogen auf das Gesamtgewicht der wässrigen Zusammensetzung. Insbesondere die in diesem Absatz offenbarten Entschäumeranteile haben sich als besonders vorteilhaft im Hinblick auf eine Unterdrückung oder Abschwächung einer auf die Dihydrotriazinverbindung der allgemeinen Formel I oder das Tautomer davon oder das Salz davon zurückgehenden Schaumbildung herausgestellt.

In weiterer Ausgestaltung der Erfindung weist die wässrige Zusammensetzung ferner ein Tensid, insbesondere nichtionisches Tensid und/oder zwitterionisches Tensid, mit der Maßgabe auf, dass das Tensid, insbesondere nichtionische Tensid und/oder zwitterionisches Tensid, und der Entschäumer voneinander verschieden gewählt sind, d.h. unterschiedliche Verbindungen darstellen.

Unter dem Ausdruck "nichtionisches Tensid" soll im Sinne der vorliegenden Erfindung ein Tensid bezeichnet werden, welches keine dissoziierbaren funktionellen Gruppen enthält und sich daher in Wasser oder einer wässrigen Flüssigkeit nicht in Ionen auftrennt.

Ein nichtionisches oder zwitterionisches Tensid hat insbesondere den Vorteil, dass keine oder wenig Interaktionen mit der Dihydrotriazinverbindung auftreten, und es darüber hinaus sehr gut verträglich gegenüber Schleimhaut und Wunden ist.

Das nichtionische Tensid ist vorzugsweise ausgewählt aus der Gruppe bestehend aus Poloxamer, Fettalkoholalkoxylat wie Fettalkoholethoxylat, Polyvinylpyrrolidon, Alkylpolyglucosid und Kombinationen, insbesondere Mischungen, von wenigstens zwei der genannten nichtionischen Tenside. Die in diesem Absatz genannten und im Folgenden näher beschriebenen nichtionischen Tenside haben sich als besonders geeignet herausgestellt, ein unter wundtherapeutischen Gesichtspunkten nachteiliges Irritationspotential der Dihydrotriazinverbindung der allgemeinen Formel I oder des Tautomers davon oder des Salzes davon zu reduzieren oder zu vermeiden.

Bevorzugt weist das Poloxamer 2 bis 130 Struktureinheiten -CH₂-CH₂-O- und/oder 15 bis 67 Struktureinheiten -CHCH₃-CH₂-O- pro Molekül auf.

Bei dem Poloxamer kann es sich insbesondere um Poloxamer 407, Poloxamer 188 oder um eine Kombination, insbesondere Mischung, davon handeln.

Die Verwendung eines Poloxamers als Tensid kann erfindungsgemäß besonders bevorzugt sein, da Poloxamere neben tensidischen Eigenschaften auch schaumdämpfende oder schaumreduzierende Eigenschaften und/oder emulgatorisch wirksame Eigenschaften besitzen. Dadurch kann mit besonderem Vorteil eine auf die Dihydrotriazinverbindung der allgemeinen Formel I oder das Tautomer davon oder das Salz davon zurückgehende Schaumbildung zusätzlich gedämpft, abgemildert oder unterdrückt und/oder eine homogene Verteilung des Entschäumers innerhalb der wässrigen Zusammensetzung erzielt oder verbessert werden.

Die Verwendung von Polyvinylpyrrolidon als Tensid kann erfindungsgemäß ebenfalls besonders bevorzugt sein, da dieses Tensid neben tensidischen Eigenschaften emulgatorisch wirksame Eigenschaften besitzt. Die im vorherigen Absatz weiter erwähnten Vorteile gelten sinngemäß.

Zusätzlich hat die Verwendung von Polyvinylpyrrolidon den Vorteil, dass es Wasser binden und mithin als Viskositätsregulator wirken kann.

Unter dem Ausdruck "Fettalkoholalkoxylat" soll im Sinne der vorliegenden Erfindung ein nichtionisches Tensid verstanden werden, dessen lipophiler Teil einen Fettalkohol aufweist oder aus einem Fettalkohol besteht und dessen hydrophiler Teil ein Polyalkylenglykol, insbesondere kurzkettiges Polyalkylenglykol, aufweist oder aus einem Polyalkylenglykol, insbesondere kurzkettigen Polyalkylenglykol, besteht. Bei dem Fettalkohol kann es sich insbesondere um einen von Capryl-, Caprin-, Laurin-, Palmitin-, Stearin- oder Ölsäure abgeleiteten Alkohol oder um verzweigte Isononyl-, Isoundecyl-, Isotridecyl-, Isopentadecyl- oder Isononadecylalkohole handeln. Das Fettalkoholalkoxylat kann im Sinne der vorliegenden Erfindung auch als Polyalkylenglykolether bezeichnet werden.

Wie bereits erwähnt, kann es sich bei dem Fettalkoholalkoxylat insbesondere um ein Fettalkoholethoxylat handeln.

Bevorzugt handelt es sich bei dem Fettalkoholethoxylat um ein Polyoxyethylenether des Laurylalkohols, ein Polyoxyethylenether des Myristylalkohols, ein Polyoxyethylenether des Cetylalkohols, ein Polyoxyethylenether des Cetylstearylalkohols, ein Polyoxyethylenether des Stearylalkohols, ein Polyoxyethylenether des Oleylalkohols, ein Polyoxyethylenether des Isononanalkohols, ein Polyoxyethylenether des Isoundecanalkohols, ein Polyoxyethylenether des Isotridecanalkohols, ein Polyoxyethylenether des Isopentadecanalkohols, ein Polyoxyethylenether des Isoheptadecanalkohols, ein Polyoxyethylenether des Isononadecanalkohols oder um eine Kombination, insbesondere Mischung, von wenigstens zwei der genannten Polyoxyethylenether.

Das Fettalkoholethoxylat kann insbesondere ausgewählt sein aus der Gruppe bestehend aus Polxoxyethylen (4) laurylether, Polxoxyethylen (7) laurylether, Polyoxyethylen (9) laurylether, Polyoxyethylen (23) laurylether, Polyoxyethylen (2) cetylether, Polyoxyethylen (10) cetylether, Polyoxyethylen (20) cetylether, Polyoxyethylen (6) cetylstearylether, Polyoxyethylen (20) cetylstearylether, Polyoxyethylen (25) cetylstearylether, Polyoxyethylen (2) stearylether, Polyoxyethylen (10) stearylether, Polyoxyethylen (20) stearylether, Polyoxyethylen (2) oleylether, Polyoxyethylen (10) oleylether, Polyoxyethylen (20) oleylether, Polyoxyethylen (10) monodecylether, Polyoxyethylen (10) tridecylether und Kombinationen, insbesondere Mischungen, von wenigstens zwei der genannten Fettalkoholethoxylate.

Mit anderen Worten kann das Fettalkoholethoxylat insbesondere ausgewählt sein aus der Gruppe bestehend aus Laureth-4, Laureth-7, Laureth-9, Laureth-23, Ceteth-2, Ceteth-10, Ceteth-20, Ceteareth-6, Ceteareth-20, Ceteareth-25, Steareth-2, Steareth-10, Steareth-20, Oleth-2, Oleth-10, Oleth-20, Deceth-10, Trideceth-10 und Kombinationen, insbesondere Mischungen, von wenigstens zwei der genannten Fettalkoholethoxylate.

Fettalkoholalkoxylate, insbesondere Fettalkoholethoxylate, haben den Vorteil, dass sie nicht nur tensidische Eigenschaften aufweisen, sondern zusätzlich auch emulgatorisch wirksam sind.

Unter dem Ausdruck "Alkylpolyglucosid" soll im Sinne der vorliegenden Erfindung ein nichtionisches Zuckertensid verstanden werden, welches einen oder mehrere Glucosebausteine und einen Alkylrest, insbesondere langkettigen Alkylrest, aufweist oder aus einem oder mehreren Glucosebausteinen und einem Alkylrest, insbesondere langkettigen Alkylrest, besteht. Der Glucosebaustein bzw. die Glucosebausteine fungieren als hydrophiler Bestandteil, während der Alkylrest die hydrophobe Gruppe darstellt.

Das Alkylpolyglucosid weist vorzugsweise 1 bis 5 Glucosebausteine und/oder einen Alkylrest mit 6 Kohlenstoffatomen bis 20 Kohlenstoffatomen, insbesondere 6 Kohlenstoffatomen bis 16 Kohlenstoffatomen, bevorzugt 8 Kohlenstoffatomen bis 14 Kohlenstoffatomen, auf.

Bevorzugt handelt es sich bei dem Alkylpolyglucosid um eine C₈- bis C₂₀-Alkylpolyglucose, insbesondere C₈- bis C₁₆-Alkylpolyglucose.

Besonders bevorzugt handelt es sich bei dem Alkylpolyglucosid um Laurylpolyglucose, Decylpolyglucose, Cocoylpolyglucose oder um eine Mischung von wenigstens zwei der genannten Alkylpolyglucoside. Der Alkylrest der Decylpolyglucose weist dabei bevorzugt 8 Kohlenstoffatome bis 16 Kohlenstoffatome, insbesondere 10 Kohlenstoffatome, auf. Der Alkylrest der Laurylpolyglucose weist bevorzugt 12 Kohlenstoffatome bis 16 Kohlenstoffatome, insbesondere 12 Kohlenstoffatome, auf. Der Alkylrest der Cocoylpolyglucose weist bevorzugt 8 Kohlenstoffatome bis 16 Kohlenstoffatome auf.

Auch Alkylpolyglucoside haben den Vorteil, dass sie nicht nur über tensidische Eigenschaften verfügen, sondern zusätzlich emulgatorisch wirksame Verbindungen darstellen.

In weiterer Ausgestaltung der Erfindung handelt es sich bei dem Tensid um ein zwitterionisches Tensid.

Unter dem Ausdruck "zwitterionisches Tensid" soll im Sinne der vorliegenden Erfindung ein Tensid bezeichnet werden, welches sowohl eine negativ als auch eine positiv geladene funktionelle Gruppe besitzt (sogenanntes amphoteres Tensid).

Das zwitterionische Tensid ist vorzugsweise ein Alkylamidoalkylbetain, insbesondere ein Alkylamidoethylbetain, Alkylamidopropylbetain oder eine Kombination, insbesondere Mischung, davon.

Bei dem Alkylamidoalkylbetain handelt es sich besonders bevorzugt um ein Alkylamidoalkylbetain einer Fettsäure. Bevorzugt weist die Fettsäure dabei 8 bis 18 Kohlenstoffatome auf. Bei der Fettsäure kann es sich um eine gesättigte oder ungesättigte Fettsäure handeln. Bevorzugt handelt es sich bei der Fettsäure um Caprylsäure, Caprinsäure, Undecylensäure (Undec-10-ensäure), Undecylsäure (n-Undecansäure), Laurinsäure, Stearinsäure, Ricinolsäure oder Kokosfettsäure.

Bevorzugt ist das Alkylamidoalkylbetain ausgewählt aus der Gruppe bestehend aus Caprylamidoalkylbetain, Caprinamidoalkylbetain, Undecylenamidoalkylbetain, Undecylamidoalkylbetain, Lauramidoalkylbetain, Lauryldimethylaminoessigsäurebetain, Stearinamidoalkylbetain, Ricinolamidoalkylbetain, Cocamidoalkylbetain und Kombinationen, insbesondere Mischungen, von wenigstens zwei der genannten Alkylamidoalkylbetaine.

Das Alkylamidoethylbetain ist bevorzugt ein Alkylamidoethylbetain auf Basis einer Fettsäure, insbesondere einer Fettsäure mit 8 bis 18 Kohlenstoffatomen. Bei der Fettsäure kann es sich um eine gesättigte oder ungesättigte Fettsäure handeln. Bevorzugt handelt es sich bei der Fettsäure um Caprylsäure, Caprinsäure, Undecylensäure (Undec-10-ensäure), Undecylsäure (n-Undecansäure), Laurinsäure, Stearinsäure, Ricinolsäure oder Kokosfettsäure.

Besonders bevorzugt ist das Alkylamidoethylbetain ausgewählt aus der Gruppe bestehend aus Caprylamidoethylbetain, Caprinamidoethylbetain, Undecylenamidoethylbetain, Undecylamidoethylbetain, Lauramidoethylbetain, Cocamidoethylbetain, Stearinamidoethylbetain, Ricinolamidoethylbetain und Kombinationen, insbesondere Mischungen, von wenigstens zwei der genannten Alkylamidoethylbetaine.

Bei dem Alkylamidopropylbetain handelt es sich bevorzugt um ein Alkylamidopropylbetain auf Basis einer Fettsäure, insbesondere einer Fettsäure mit 8 bis 18 Kohlenstoffatomen. Bei der Fettsäure kann es sich um eine gesättigte oder ungesättigte Fettsäure handeln. Bevorzugt handelt es sich bei der Fettsäure um Caprylsäure, Caprinsäure, Undecylensäure (Undec-10-ensäure), Undecylsäure (n-Undecansäure), Laurinsäure, Stearinsäure, Ricinolsäure oder Kokosfettsäure.

Besonders bevorzugt ist das Alkylamidopropylbetain ausgewählt aus der Gruppe bestehend aus Caprylamidopropylbetain, Caprinamidopropylbetain, Undecylenamidopropylbetain, Undecylamidopropylbetain, Cocamidopropylbetain, Stearinamidopropylbetain, Ricinolamidopropylbetain und Kombinationen, insbesondere Mischungen, von wenigstens zwei der genannten Alkylamidopropylbetaine.

Bei dem optional vorgesehenen Tensid kann es sich weiterhin auch um eine Kombination, insbesondere Mischung, der in den vorherigen Absätzen beschriebenen Tenside, insbesondere um eine Kombination, insbesondere Mischung, eines nichtionischen Tensids und eines zwitterionischen Tensids, handeln. Insoweit wird vollständig auf die in den bisherigen Absätzen beschriebenen Tenside Bezug genommen.

Weiterhin kann es sich bei dem Tensid um ein Poloxamer handeln, wobei die wässrige Zusammensetzung abgesehen von dem Tensid keine entschäumende Verbindung und/oder keine emulgatorisch wirksame Verbindung aufweist. Aufgrund der bereits erwähnten entschäumenden und/oder emulgatorisch wirksamen Eigenschaften eines Poloxamers kann die zusätzliche Verwendung eines Entschäumers und/oder Emulgators mit besonderem Vorteil entbehrlich sein. Bezüglich weiterer Merkmale und Vorteile des Poloxamers wird vollständig auf die bisherige Beschreibung Bezug genommen.

Weiterhin kann es sich bei dem Tensid um Polyvinylpyrrolidon handeln, wobei die wässrige Zusammensetzung abgesehen von dem Tensid keine entschäumende Verbindung und/oder keine emulgatorisch wirksame Verbindung und/oder keine wasserbindende Verbindung aufweist. Aufgrund der bereits erwähnten entschäumenden und/oder emulgatorisch wirksamen und/oder verdickenden, d.h. wasserbindenden, Eigenschaften von Polyvinylpyrrolidon kann die zusätzliche Verwendung eines Entschäumers und/oder Emulgators und/oder Verdickungsmittels mit besonderem Vorteil entbehrlich sein. Bezüglich weiterer Merkmale und Vorteile von Polyvinylpyrrolidon wird vollständig auf die bisherige Beschreibung Bezug genommen.

Weiterhin kann es sich bei dem Tensid um ein Fettalkoholalkoxylat, insbesondere Fettalkoholethoxylat, und/oder Alkylpolyglucosid handeln, wobei die wässrige Zusammensetzung abgesehen von dem Tensid keine emulgatorisch wirksame Verbindung aufweist. Aufgrund der bereits erwähnten emulgatorisch wirksamen Eigenschaften eines Fettalkoholalkoxylats, insbesondere Fettalkoholethoxylats, und/oder Alkylpolyglucosids kann die zusätzliche Verwendung eines Emulgators mit besonderem Vorteil entbehrlich sein. Bezüglich weiterer Merkmale und Vorteile des Fettalkoholalkoxylats, insbesondere Fettalkoholethoxylats, und/oder Alkylpolyglucosids wird vollständig auf die bisherige Beschreibung Bezug genommen.

In weiterer Ausgestaltung der Erfindung weist das Tensid einen Anteil, insbesondere Aktiv-Anteil, von 0.01 Gew.-% bis 10.0 Gew.-%, insbesondere 0.05 Gew.-% bis 2.0 Gew.-%, vorzugsweise 0.1 Gew.-% bis 1.0 Gew.-%, auf, bezogen auf das Gesamtgewicht der wässrigen Zusammensetzung. Insbesondere die in diesem Absatz offenbarten Tensidanteile sind in besonderer Weise dazu geeignet, eine irritierende Wirkung der Dihydrotriazinverbindung der allgemeinen Formel I oder des Tautomers davon oder des Salzes davon signifikant zu reduzieren.

In weiterer Ausgestaltung der Erfindung weist die wässrige Zusammensetzung ferner einen Emulgator mit der Maßgabe auf, dass der Emulgator und ein optional vorhandenes Tensid voneinander verschieden gewählt sind, d.h. unterschiedliche Verbindungen darstellen. Der Emulgator ist vorzugsweise ausgewählt aus der Gruppe bestehend aus Alkoholethoxylat, Alkylpolyglucosid, Polysorbat, ethoxyliertes Rizinusöl und Kombinationen, insbesondere Mischungen, von wenigstens zwei der genannten Emulgatoren. Bezüglich weiterer Merkmale und Vorteile des Tensids wird vollständig auf die bisherige Beschreibung Bezug genommen.

Bei dem Alkoholethoxylat handelt es sich vorzugsweise um ein Fettalkoholethoxylat.

Das Fettalkoholethoxylat kann insbesondere ein Polyoxyethylenether des Laurylalkohols, ein Polyoxyethylenether des Myristylalkohols, ein Polyoxyethylenether des Isononylalkohols, ein Polyoxyethylenether des Isoundecylalkohols, ein Polyoxyethylenether des Isotridecylylalkohols, ein Polyoxyethylenether des Cetylalkohols, ein Polyoxyethylenether des Cetylstearylalkohols, ein Polyoxyethylenether des Stearylalkohols, ein Polyoxyethylenether des Oleylalkohols oder eine Kombination, insbesondere Mischung, von wenigstens zwei der genannten Polyoxyethylenether sein.

Bevorzugt ist das Alkoholethoxylat ausgewählt aus der Gruppe bestehend aus Polxoxyethylen (4) laurylether, Polxoxyethylen (7) laurylether, Polyoxyethylen (9) laurylether, Polyoxyethylen (23) laurylether, Polyoxyethylen (2) cetylether, Polyoxyethylen (10) cetylether, Polyoxyethylen (20) cetylether, Polyoxyethylen (6) cetylstearylether, Polyoxyethylen (20) cetylstearylether, Polyoxyethylen (25) cetylstearylether, Polyoxyethylen (2) stearylether, Polyoxyethylen (10) stearylether, Polyoxyethylen (20) stearylether, Polyoxyethylen (2) oleylether, Polyoxyethylen (10) oleylether, Polyoxyethylen (20) oleylether, Polyoxyethylen (10) monodecylether, Polyoxyethylen (10) tridecylether und Kombinationen, insbesondere Mischungen, von wenigstens zwei der genannten Fettalkoholethoxylate.

Mit anderen Worten kann das Alkoholethoxylat bevorzugt ausgewählt sein aus der Gruppe bestehend aus Laureth-4, Laureth-7, Laureth-9, Laureth-23, Ceteth-2, Ceteth-10, Ceteth-20, Ceteareth-6, Ceteareth-20, Ceteareth-25, Steareth-2, Steareth-10, Steareth-20, Oleth-2, Oleth-10, Oleth-20, Deceth-10, Trideceth-10 und Kombinationen, insbesondere Mischungen, von wenigstens zwei der genannten Fettalkoholethoxylate.

Das Alkylpolyglucosid weist vorzugsweise 1 bis 5 Glucosebausteine und/oder einen Alkylrest mit 8 Kohlenstoffatomen bis 20 Kohlenstoffatomen, insbesondere 8 Kohlenstoffatomen bis 16 Kohlenstoffatomen, bevorzugt 8 Kohlenstoffatomen bis 14 Kohlenstoffatomen, auf.

Bevorzugt handelt es sich bei dem Alkylpolyglucosid um eine C₈- bis C₂₀-Alkylpolyglucose, insbesondere C₈- bis C₁₆-Alkylpolyglucose.

Besonders bevorzugt handelt es sich bei dem Alkylpolyglucosid um Laurylpolyglucose, Decylpolyglucose, Cocoylpolyglucose oder um eine Mischung von wenigstens zwei der genannten Alkylpolyglucoside. Der Alkylrest der Decylpolyglucose weist dabei bevorzugt 8 Kohlenstoffatome bis 16 Kohlenstoffatome, insbesondere 10 Kohlenstoffatome, auf. Der Alkylrest der Laurylpolyglucose weist bevorzugt 12 Kohlenstoffatome bis 16 Kohlenstoffatome, insbesondere 12 Kohlenstoffatome, auf. Der Alkylrest der Cocoylpolyglucose weist bevorzugt 8 Kohlenstoffatome bis 16 Kohlenstoffatome auf.

Unter dem Ausdruck "Polysorbat" soll im Sinne der vorliegenden Erfindung ein ethoxylierter Sorbitansäurefettsäureester verstanden werden.

Das Polysorbat kann insbesondere ausgewählt sein aus der Gruppe bestehend aus Polyoxyethylen-(20)-sorbitanmonolaurat, Polyoxyethylen-(4)-sorbitanmonolaurat, Polyoxyethylen-(20)-sorbitanmonopalmitat, Polyoxyethylen-(20)-sorbitanmonostearat, Polyoxyethylen-(4)-sorbitanmonostearat, Polyoxyethylen-(20)-sorbitantristearat, Polyoxyethylen-(20)-sorbitanmonooleat, Polyoxyethylen-(5)-sorbitanmonooleat, Polyoxyethylen-(20)-sorbitantrioleat, Polyoxyethylen-(20)-sorbitanmonoisostearat und Kombinationen, insbesondere Mischungen, von wenigstens zwei der genannten Polysorbate.

Mit anderen Worten kann das Polysorbat insbesondere ausgewählt sein aus der Gruppe bestehend aus Polysorbat 20, Polysorbat 21, Polysorbat 40, Polysorbat 60, Polysorbat 61, Polysorbat 65, Polysorbat 80, Polysorbat 81, Polysorbat 85, Polysorbat 120 und Kombinationen, insbesondere Mischungen, von wenigstens zwei der genannten Polysorbate.

In weiterer Ausgestaltung der Erfindung weist der Emulgator einen Anteil von 0.001 Gew.-% bis 1,5 Gew.-%, insbesondere 0.01 Gew.-% bis 1.5 Gew.-%, insbesondere 0.02 Gew.-% bis 1.0 Gew.-%, vorzugsweise 0.05 Gew.-% bis 0.5 Gew.-%, auf, bezogen auf das Gesamtgewicht der wässrigen Zusammensetzung. Insbesondere die in diesem Absatz offenbarten Emulgatoranteile haben sich als besonders vorteilhaft im Hinblick auf eine homogene Verteilung des Entschäumers innerhalb der wässrigen Zusammensetzung herausgestellt.

In weiterer Ausgestaltung der Erfindung weist die wässrige Zusammensetzung ferner einen Zusatzstoff auf, welcher ausgewählt ist aus der Gruppe bestehend aus Komplexbildner, Feuchthaltemittel, Säure, Alkali, organisches Lösungsmittel und Kombinationen, insbesondere Mischungen, von wenigstens zwei der genannten Zusatzstoffe.

Der Komplexbildner kann insbesondere ausgewählt sein aus der Gruppe bestehend aus Citronensäure, Weinsäure, Bernsteinsäure, Methylglycindiacetat, Ethyldiamintetraacetat, N,N'-Bis-(carboxymethyl)-L-glutamat, Polyasparaginsäure, Iminodisuccinat, Salze der genannten Komplexbildner und Kombinationen, insbesondere Mischungen, von wenigstens zwei der genannten Komplexbildner.

Weiterhin kann der Komplexbildner einen Anteil von 0.01 Gew.-% bis 10.0 Gew.-%, insbesondere 0.02 Gew.-% bis 2.0 Gew.-%, vorzugsweise 0.05 Gew.-% bis 1.0 Gew.-%, aufweisen, bezogen auf das Gesamtgewicht der wässrigen Zusammensetzung.

Das Feuchthaltemittel kann ausgewählt sein aus der Gruppe bestehend aus Glycerin, Polydextrose, Sorbit, Ethylenglykol, Polyethylenglykol, Propylenglykol, Butylenglykol, Pentylenglykol, Hexandiol, Octandiol, Glucose, Fructose, Glucuronsäure, Lactose, Milchsäure, Lactat, Lactulose, Saccharose, Hyaluronsäure, Xylitol, Xylose und Kombinationen, insbesondere Mischungen, von wenigstens zwei der genannten Feuchthaltemittel.

Weiterhin kann das Feuchthaltemittel einen Anteil von 0.05 Gew.-% bis 10.0 Gew.-%, insbesondere 0.1 Gew.-% bis 7.5 Gew.-%, vorzugsweise 0.5 Gew.-% bis 5 Gew.-%, aufweisen, bezogen auf das Gesamtgewicht der wässrigen Zusammensetzung.

Die Säure kann ausgewählt sein aus der Gruppe bestehend aus Ameisensäure, Essigsäure, Propionsäure, Milchsäure, Buttersäure, Isobuttersäure, Äpfelsäure, Maleinsäure, Malonsäure, Fumarsäure, Bernsteinsäure, Bernsteinsäuremonoamid, Glutarsäure, Weinsäure, Oxalsäure, Citronensäure, Glykolsäure, Glucuronsäure, Ascorbinsäure, Asparaginsäure, Glutaminsäure, Benzoesäure, Phthalsäure, Salicylsäure, Anthranilsäure, Benzolsulfonsäure, p-Toluolsulfonsäure, Methansulfonsäure und Kombinationen, insbesondere Mischungen, von wenigstens zwei der genannten Säuren.

Weiterhin kann die Säure einen Anteil von 0.01 Gew.-% bis 10.0 Gew.-%, insbesondere 0.1 Gew.-% bis 6.0 Gew.-%, vorzugsweise 0.1 Gew.-% bis 5.0 Gew.-%, aufweisen, bezogen auf das Gesamtgewicht der wässrigen Zusammensetzung.

Die Alkalien können ausgewählt sein aus der Gruppe bestehend aus Natriumhydroxid, Kaliumhydroxid, Calciumhydroxid und Magnesiumhydroxid und Kombinationen, insbesondere Mischungen, von wenigstens zwei der genannten Alkalien.

Weiterhin können die Alkalien einen Anteil von 0.01 Gew.-% bis 10.0 Gew.-%, insbesondere 0.1 Gew.-% bis 6.0 Gew.-%, vorzugsweise 0.1 Gew.-% bis 5.0 Gew.-%, aufweisen, bezogen auf das Gesamtgewicht der wässrigen Zusammensetzung.

Das organische Lösungsmittel kann ausgewählt sein aus der Gruppe bestehend aus Ethanol, Propan-1-ol, Propan-2-ol und Kombinationen, insbesondere Mischungen, von wenigstens zwei der genannten organischen Lösungsmittel.

Weiterhin kann das organische Lösungsmittel einen Anteil von 0.1 Gew.-% bis 10.0 Gew.-%, insbesondere 0.5 Gew.-% bis 10.0 Gew.-%, vorzugsweise 1.0 Gew.-% bis 10.0 Gew.-%, aufweisen, bezogen auf das Gesamtgewicht der wässrigen Zusammensetzung.

Bevorzugt weist die wässrige Zusammensetzung einen Anteil an Wasser von > 80 Gew.-%, insbesondere > 90 Gew.-%, auf, bezogen auf das Gesamtgewicht der wässrigen Zusammensetzung.

In weiterer Ausgestaltung der Erfindung handelt es sich bei der wässrigen Zusammensetzung um eine wässrige Zusammensetzung zur Anwendung oder Verwendung bei der Vorbeugung oder Behandlung von Schleimhaut und/oder Wunden, insbesondere akuten oder chronischen Wunden, und/oder zur Anwendung oder Verwendung bei der Vorbeugung oder Behandlung von Infektionen, insbesondere durch Pilze, insbesondere Hefen, verursachte oder mitverursachte Infektionen, und/oder zur Anwendung oder Verwendung bei der Vorbeugung oder Behandlung von Infektionskrankheiten, insbesondere durch Pilze, insbesondere Hefen, verursachte oder mitverursachte Infektionskrankheiten. Bei den Infektionskrankheiten kann es sich beispielsweise um Candidose, Wundrose oder ein Erysipel handeln.

Weitere Merkmale und Vorteile der Erfindung ergeben sich aus der nachfolgenden Beschreibung bevorzugter Ausführungsbeispiele. Dabei können einzelne Merkmale jeweils für sich alleine oder in Kombination miteinander verwirklicht sein. Die beschriebenen Ausführungsbeispiele dienen lediglich der weiteren Erläuterung der Erfindung, ohne diese hierauf zu beschränken.

### BEISPIELTEIL

### 1. Herstellung einer erfindungsgemäßen Wundspüllösung

In einem geeigneten Mischreaktor wurden 9912.5 g Wasser vorgelegt. Danach wurde ein Rührwerk derart eingestellt, dass sich an der Wasseroberfläche ein leichter Torus bildete. Anschließend wurden 25.00 g Tween 20 (Polysorbat 20) sowie 2.50 g N-(2-Ethylhexyl)-Isononanamid hinzugegeben. Dann wurden 50.00 g Cocamidopropylbetain (50%) hinzugegeben. Danach wurden 10.00 g Femotaxidine hinzugegeben. Anschließend wurde das Gemisch für eine Stunde gerührt. Die hierbei erhaltene Lösung wies die nachfolgende in Tabelle 1 wiedergegebene Zusammensetzung auf:

**Tabelle 1: Beispiel für eine erfindungsgemäße Wundspüllösung**

| | |
|---|---|
| Femotaxidine | 0.100 Gew.-% |
| Gereinigtes Wasser | 99.125 Gew.-% |
| Cocamidopropylbetain (50%) | 0.500 Gew.-% |
| Tween 20 | 0.250 Gew.-% |
| Alkylamid | 0.025 Gew.-% |

### 2. Herstellung einer weiteren erfindungsgemäßen Wundspüllösung

In einem geeigneten Mischreaktor wurden 9889.0 g Wasser vorgelegt. Danach wurde ein Rührwerk derart eingestellt, dass sich an der Wasseroberfläche ein leichter Torus bildete. Anschließend wurden 40.00 g Tween 20 sowie 1.00 g Polyether-Siloxan zugegeben. Danach wurden 50.00 g Cocamidopropylbetain (50%) zugegeben. Schließlich wurden 20.00 g Femotaxidine zugegeben. Das Gemisch wurde für eine Stunde gerührt. Die hierbei erhaltene Lösung wies die nachfolgende in Tabelle 2 wiedergegebene Zusammensetzung auf:

**Tabelle 2: Beispiel für eine erfindungsgemäße Wundspüllösung**

| | |
|---|---|
| Femotaxidine | 0.200 Gew.-% |
| Gereinigtes Wasser | 98.890 Gew.-% |
| Cocamidopropylbetain (50%) | 0.500 Gew.-% |
| Tween 20 | 0.400 Gew.-% |
| Silikon | 0.010 Gew.-% |

### 3. Herstellung einer weiteren erfindungsgemäßen Wundspüllösung

In einem geeigneten Mischreaktor wurden 9885.0 g Wasser vorgelegt. Danach wurde ein Rührwerk derart eingestellt, dass sich an der Wasseroberfläche ein leichter Torus bildete. Anschließend wurden 17.50 g Laureth-7 sowie 2.50 g N-(2-Ethylhexyl)-Isononanamid zugegeben. Danach wurden 90.00 g Undecylenamidopropylbetain (35%) zugegeben. Schließlich wurden 5.00 g Femotaxidine zugegeben. Das Gemisch wurde für eine Stunde gerührt. Die hierbei erhaltene Lösung wies die nachfolgende in Tabelle 3 wiedergegebene Zusammensetzung auf:

**Tabelle 3: Beispiel für eine weitere erfindungsgemäße Wundspüllösung**

| | |
|---|---|
| Femotaxidine | 0.050 Gew.-% |
| Gereinigtes Wasser | 98.850 Gew.-% |
| Undecylenamidopropylbetain (35%) | 0.900 Gew.-% |
| Laureth-7 | 0.175 Gew.-% |
| Alkylamid | 0.025 Gew.-% |

### 4. Herstellung einer weiteren erfindungsgemäßen Wundspüllösung

In einem geeigneten Mischreaktor wurden 9833.0 g Wasser vorgelegt. Danach wurde ein Rührwerk derart eingestellt, dass sich an der Wasseroberfläche ein leichter Torus bildete. Anschließend wurden 15.00 g Laureth-7 sowie 2.00 g Polyether-Siloxan zugegeben. Danach wurden 125.0 g Undecylenamidopropylbetain (35%) zugegeben. Schließlich wurden 25.00 g Femotaxidine zugegeben. Das Gemisch wurde für eine Stunde gerührt. Die hierbei erhaltene Lösung wies die nachfolgende in Tabelle 4 wiedergegebene Zusammensetzung auf:

**Tabelle 4: Beispiel für eine weitere erfindungsgemäße Wundspüllösung**

| | |
|---|---|
| Femotaxidine | 0.250 Gew.-% |
| Gereinigtes Wasser | 98.330 Gew.-% |
| Undecylenamidopropylbetain (35%) | 1.250 Gew.-% |
| Laureth-7 | 0.150 Gew.-% |
| Silikon | 0.020 Gew.-% |

### 5. Herstellung einer weiteren erfindungsgemäßen Wundspüllösung

In einem geeigneten Mischreaktor wurden 9904.4 g Wasser vorgelegt. Danach wurde ein Rührwerk derart eingestellt, dass sich an der Wasseroberfläche ein leichter Torus bildete. Anschließend wurden 15.00 g Laureth-7, 0.50 g N-(2-Ethylhexyl)-Isononanamid sowie 0.10 g 3D-modifizierte-"Crosslink"-Siloxane zugegeben. Danach wurden 70.00 g Caprylamidopropylbetain (35%) zugegeben. Schließlich wurden 10.00 g Femotaxidine zugegeben. Das Gemisch wurde für eine Stunde gerührt. Die hierbei erhaltene Lösung wies die nachfolgende in Tabelle 5 wiedergegebene Zusammensetzung auf:

**Tabelle 5: Beispiel für eine weitere erfindungsgemäße Wundspüllösung**

| | |
|---|---|
| Femotaxidine | 0.100 Gew.-% |
| Gereinigtes Wasser | 99.044 Gew.-% |
| Alkylamidopropylbetain (35%) | 0.700 Gew.-% |
| Laureth-7 | 0.150 Gew.-% |
| Alkylamid | 0.005 Gew.-% |
| Silikon | 0.001 Gew.-% |

### 6. Herstellung einer weiteren erfindungsgemäßen Wundspüllösung

In einem geeigneten Mischreaktor wurden 9889.5 g Wasser vorgelegt. Danach wurde ein Rührwerk derart eingestellt, dass sich an der Wasseroberfläche ein leichter Torus bildete. Anschließend wurden 20.00 g Poloxamer 188 sowie 0.50 g 3D-modifizierte-"Crosslink"-Siloxane zugegeben. Danach wurden 50.00 g Cocamidopropylbetain (50%) zugegeben. Schließlich wurden 10.00 g Femotaxidine zugegeben. Das Gemisch wurde für eine Stunde gerührt. Die hierbei erhaltene Lösung wies die nachfolgende in Tabelle 6 wiedergegebene Zusammensetzung auf:

**Tabelle 6: Beispiel für eine weitere erfindungsgemäße Wundspüllösung**

| | |
|---|---|
| Femotaxidine | 0.100 Gew.-% |
| Gereinigtes Wasser | 98.895 Gew.-% |
| Cocamidopropylbetain (50%) | 0.800 Gew.-% |
| Poloxamer | 0.200 Gew.-% |
| Silikon | 0.005 Gew.-% |

### 7. Herstellung einer weiteren erfindungsgemäßen Wundspüllösung

In einem geeigneten Mischreaktor wurden 9958.0 g Wasser vorgelegt. Danach wurde ein Rührwerk derart eingestellt, dass sich an der Wasseroberfläche ein leichter Torus bildete. Anschließend wurden 30.00 g Tween 20 sowie 2.00 g N-(2-Ethylhexyl)-Isononanamid hinzugegeben. Danach wurden 10.00 g Femotaxidine hinzugegeben. Anschließend wurde das Gemisch für eine Stunde gerührt. Die hierbei erhaltene Lösung wies die nachfolgende in Tabelle 7 wiedergegebene Zusammensetzung auf:

**Tabelle 7: Beispiel für eine weitere erfindungsgemäße Wundspüllösung**

| | |
|---|---|
| Femotaxidine | 0.100 Gew.-% |
| Gereinigtes Wasser | 99.580 Gew.-% |
| Tween 20 | 0.300 Gew.-% |
| Alkylamid | 0.020 Gew.-% |

### 8. Herstellung einer weiteren erfindungsgemäßen Wundspüllösung

In einem geeigneten Mischreaktor wurden 9966.4 g Wasser vorgelegt. Danach wurde ein Rührwerk derart eingestellt, dass sich an der Wasseroberfläche ein leichter Torus bildete. Anschließend wurden 12.50 g Laureth-7, 1.00 g N-(2-Ethylhexyl)-Isononanamid sowie 0.10 g 3D-modifizierte-"Crosslink"-Siloxane hinzugegeben. Danach wurden 20.00 g Femotaxidine hinzugegeben. Anschließend wurde das Gemisch für eine Stunde gerührt. Die hierbei erhaltene Lösung wies die nachfolgende in Tabelle 8 wiedergegebene Zusammensetzung auf:

**Tabelle 8: Beispiel für eine weitere erfindungsgemäße Wundspüllösung**

| | |
|---|---|
| Femotaxidine | 0.200 Gew.-% |
| Gereinigtes Wasser | 99.664 Gew.-% |
| Laureth-7 | 0.125 Gew.-% |
| Alkylamid | 0.010 Gew.-% |
| Silikon | 0.001 Gew.-% |

### 9. Herstellung einer weiteren erfindungsgemäßen Wundspüllösung

In einem geeigneten Mischreaktor wurden 9820.0 g Wasser vorgelegt. Danach wurde ein Rührwerk derart eingestellt, dass sich an der Wasseroberfläche ein leichter Torus bildete. Anschließend wurden 500.00 g Polyvinylpyrrolidon hinzugegeben. Danach wurden 70.00 g Undecylenamidopropylbetain (35%) zugegeben. Danach wurden 10.00 g Femotaxidine hinzugegeben. Anschließend wurde das Gemisch für eine Stunde gerührt. Die hierbei erhaltene Lösung wies die nachfolgende in Tabelle 9 wiedergegebene Zusammensetzung auf:

**Tabelle 9: Beispiel für eine weitere erfindungsgemäße Wundspüllösung**

| | |
|---|---|
| Femotaxidine | 0.100 Gew.-% |
| Gereinigtes Wasser | 98.200 Gew.-% |
| Polyvinylpyrrolidon | 5.000 Gew.-% |
| Undecylenamidopropylbetain (35%) | 0.700 Gew.-% |

### 10. Herstellung einer weiteren erfindungsgemäßen Wundspüllösung

In einem geeigneten Mischreaktor wurden 9890.0 g Wasser vorgelegt. Danach wurde ein Rührwerk derart eingestellt, dass sich an der Wasseroberfläche ein leichter Torus bildete. Anschließend wurden 100.00 g Poloxamer 188 hinzugegeben. Danach wurden 10.00 g Femotaxidine hinzugegeben. Anschließend wurde das Gemisch für eine Stunde gerührt. Die hierbei erhaltene Lösung wies die nachfolgende in Tabelle 10 wiedergegebene Zusammensetzung auf:

**Tabelle 10: Beispiel für eine weitere erfindungsgemäße Wundspüllösung**

| | |
|---|---|
| Femotaxidine | 0.100 Gew.-% |
| Gereinigtes Wasser | 98.900 Gew.-% |
| Poloxamer | 1.000 Gew.-% |

### 11. Herstellung einer nicht erfindungsgemäßen Wundspüllösung

In einem geeigneten Mischreaktor wurden 9980.0 g Wasser vorgelegt. Danach wurde ein Rührwerk derart eingestellt, dass sich an der Wasseroberfläche ein leichter Torus bildete. Anschließend wurden 20.00 g Femotaxidine hinzugegeben. Anschließend wurde das Gemisch für eine Stunde gerührt. Die hierbei erhaltene Lösung wies die nachfolgende in Tabelle 11 wiedergegebene Zusammensetzung auf:

**Tabelle 11: Beispiel für eine nicht erfindungsgemäße Wundspüllösung**

| | |
|---|---|
| Femotaxidine | 0.200 Gew.-% |
| Gereinigtes Wasser | 99.800 Gew.-% |

Die unter 1. Bis 10. hergestellten erfindungsgemäßen Wundspüllösungen wiesen gegenüber einer wässrigen Femotaxidine-Lösung (0.1 Gew.-% oder 0.2 Gew.-% [Lösung 11]) große Unterschiede im Schaumverhalten auf. Während der Schaum bei den erfindungsgemäßen Wundspüllösungen viel mehr Energie zur Entstehung erforderte und dann innerhalb eines sehr kurzen Zeitraumes, insbesondere innerhalb weniger Minuten, zerfiel, entstand der Schaum bei der zu Vergleichszwecken herangezogenen wässrigen Femotaxidine-Lösung sehr leicht und blieb über einen Zeitraum von über 10 Stunden bestehen. Besonders die Rezepturen 5 und 8 zeigten einen sehr schnellen Schaumabbau.

Die Rezeptur 9, aber auch die Rezeptur 11 zeigten im Vergleich zu dem Handelspräparat Octenisept eine deutlich schnellere Wirksamkeit gegen Hefen gemäss EN13624 und konnten gleich noch im HET-CAM Test mit deutlich geringerem Reizpotential überzeugen.

**Keimzahlreduktion in log₁₀; Kriterium, um den Test nach EN13624 zu bestehen ist > 4.**

| EN 13624 Schmutzkonditionen | C. albicans 60 s | C. albicans 90 s | C. albicans 120 s | HET-CAM |
|---|---|---|---|---|
| Rezeptur 9 | 4.5 | > 4.5 | > 4.5 | 1 |
| Rezeptur 11 | 4.5 | > 4.5 | > 4.5 | 2 |
| Octenisept | - | - | 3.0 | 6 |

Der HET-CAM (Hühner-Ei-Test an der Chorion-Allantois-Membran) ist ein Verfahren zur Überprüfung der Schleimhautverträglichkeit von Chemikalien. Die CAM ist die Aderhaut des Eies. Für den Test wird die zu prüfende Substanz auf die CAM gebracht. Die Reaktionen der Membran (Blutungen, Veränderungen der Blutgefäße oder des Eiklars) werden beobachtet, mit einer Referenz (z.B. Natriumdodecylsulfat) verglichen und ausgewertet. Der Test ist als offizieller Vortest für die Prüfung der Schleimhautverträglichkeit anerkannt.

Im HET-CAM bedeutet eine 6 eine mässige Reizung, während Werte von 1-5 als geringe Reizung bewertet werden.

## Patentansprüche

1. Wässrige Zusammensetzung, insbesondere in Form einer wässrigen Lösung oder eines Hydrogels, aufweisend
- eine Dihydrotriazinverbindung der nachstehenden allgemeinen Formel I: 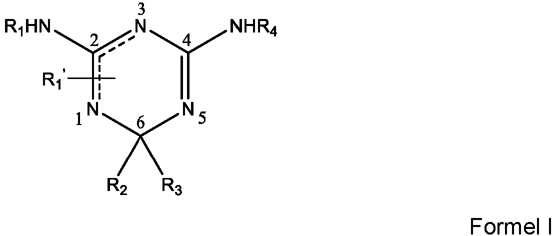 wobei
- R₁ (i) eine Phenylgruppe oder eine Phenylalkylgruppe, von denen jede optional mit 1 bis 3 Substituenten, ausgewählt aus der Gruppe bestehend aus C₁₋₆-Alkoxygruppe, Hydroxygruppe, einem Halogenatom, C₁₋₆-Halogenalkylgruppe, C₁₋₆-Alkylgruppe, einer Sulfonamidogruppe und C₁₋₆-Halogenalkoxygruppe, substituiert ist, (ii) eine Naphthylgruppe oder eine Naphthylalkylgruppe, (iii) eine heterocyclische Gruppe, eine heterocyclische Alkylgruppe oder eine heterocyclische Aminoalkylgruppe, (iv) eine Alkylgruppe mit 1 bis 16 Kohlenstoffatomen oder (v) eine Cycloalkylgruppe oder eine Cycloalkyl-alkylgruppe bedeutet,
- R₁' ein Wasserstoffatom, das an Position 1 oder 3 des Dihydrotriazinrings an das Stickstoffatom gebunden ist, bedeutet,
- R₂ und R₃ unabhängig voneinander ein Wasserstoffatom oder eine Methylgruppe bedeuten,
- R₄ eine Alkylgruppe mit 7 bis 16 Kohlenstoffatomen bedeutet und
- die Strichlinie angibt, dass die Stellung einer Doppelbindung entweder zwischen den Positionen 1 und 2 oder zwischen den Positionen 2 und 3 des Dihydrotriazinrings liegt,
oder ein Tautomer davon oder ein Salz davon und
- einen Entschäumer
zur Anwendung bei der Vorbeugung oder Behandlung von durch Pilze verursachte oder mitverursachte Infektionen und/oder Infektionskrankheiten.

2. Wässrige Zusammensetzung zur Anwendung nach Anspruch 1, **dadurch gekennzeichnet, dass** R eine Phenylgruppe oder eine Phenylalkylgruppe, insbesondere Benzylgruppe, bedeutet, von denen jede optional durch 1 bis 3 Substituenten, ausgewählt aus der Gruppe bestehend aus Fluoratom, Chloratom, Hydroxygruppe, Methylgruppe, tert-Butylgruppe, Trifluoromethylgruppe und Methoxygruppe, substituiert ist, R₁' ein Wasserstoffatom bedeutet, das an Position 1 oder 3 des Dihydrotriazinrings an das Stickstoffatom gebunden ist, R₂ und R₃ jeweils eine Methylgruppe bedeuten und R₄ eine n-Octylgruppe, n-Nonylgruppe oder n-Decylgruppe bedeutet.

3. Wässrige Zusammensetzung zur Anwendung nach Anspruch 1 oder 2, **dadurch gekennzeichnet dass** R₁ eine Phenylgruppe, 4-Chlorophenylgruppe, 2,4-Difluorophenylgruppe, 2,3,4-Trifluorophenylgruppe, 4-tert-Butylphenylgruppe, 4-Methoxyphenylgruppe, 2-Methoxy-4-tert-butylphenylgruppe, 4-Trifluoromethoxyphenylgruppe, Benzylgruppe, Methylbenzylgruppe, 4-Methylbenzylgruppe, 4-Methoxybenzylgruppe, 3,4-Dimethoxybenzylgruppe, 4-Hydroxybenzylgruppe, 3,4-Dichlorobenzylgruppe, 2,3,4-Trichlorobenzylgruppe, 4-Trifluoromethylbenzylgruppe, 1-Phenylethylgruppe, 2-Phenylethylgruppe, 1-Phenylpropylgruppe, 2-Phenylpropylgruppe oder 3-Phenylpropylgruppe, bevorzugt eine 4-Methylbenzylgruppe, bedeutet, R₁' ein Wasserstoffatom bedeutet, das an Position 1 oder 3 des Dihydrotriazinrings an das Stickstoffatom gebunden ist, R₂ und R₃ jeweils eine Methylgruppe bedeuten und R₄ eine n-Octylgruppe, n-Nonylgruppe oder n-Decylgruppe bedeutet.

4. Wässrige Zusammensetzung zur Anwendung nach einem der vorhergehenden Ansprüche, **dadurch gekennzeichnet, dass** R₁ eine Methylbenzylgruppe, vorzugsweise 4-Methylbenzylgruppe, bedeutet und/oder R₂ und R₃ jeweils eine Methylgruppe bedeuten und/oder R₄ eine n-Octylgruppe bedeutet.

5. Wässrige Zusammensetzung zur Anwendung nach einem der vorhergehenden Ansprüche, **dadurch gekennzeichnet, dass** R₁ eine Methylbenzylgruppe, vorzugsweise 4-Methylbenzylgruppe, bedeutet, R₁' ein Wasserstoffatom bedeutet, das an Position 1 oder 3 des Dihydrotriazinrings an das Stickstoffatom gebunden ist, R₂ und R₃ jeweils eine Methylgruppe bedeuten und R₄ eine n-Octylgruppe bedeutet.

6. Wässrige Zusammensetzung zur Anwendung nach einem der vorhergehenden Ansprüche, **dadurch gekennzeichnet, dass** es sich bei der Dihydrotriazinverbindung um 4-Octylamino-1,6-dihydro-6,6-dimethyl-2-(4'-methylbenzylamino)-1,3,5-triazingluconat, 4-Octylamino-3,6-dihydro-6,6-dimethyl-2-(4'-methylbenzylamino)-1,3,5-triazingluconat oder ein Tautomer davon handelt.

7. Wässrige Zusammensetzung zur Anwendung nach einem der vorhergehenden Ansprüche, **dadurch gekennzeichnet, dass** die Dihydrotriazinverbindung einen Anteil von von 0.001 Gew.-% bis 1.00 Gew.-%, insbesondere 0.01 Gew.-% bis 0.50 Gew.-%, vorzugsweise 0.025 Gew.-% bis 0.25 Gew.-%, auf, bezogen auf das Gesamtgewicht der wässrigen Zusammensetzung.

8. Wässrige Zusammensetzung zur Anwendung nach einem der vorhergehenden Ansprüche, **dadurch gekennzeichnet, dass** der Entschäumer ausgewählt ist aus der Gruppe bestehend aus Alkylamid, Silikon, Poloxamer und Kombinationen von wenigstens zwei der genannten Entschäumer.

9. Wässrige Zusammensetzung zur Anwendung nach einem der vorhergehenden Ansprüche, **dadurch gekennzeichnet, dass** der Entschäumer einen Anteil von 0.0001 Gew.-% bis 2.0 Gew.-%, insbesondere 0.0005 Gew.-% bis 1.5 Gew.-%, vorzugsweise 0.001 Gew.-% bis 1.0 Gew.-%, aufweist, bezogen auf das Gesamtgewicht der wässrigen Zusammensetzung.

10. Wässrige Zusammensetzung zur Anwendung nach einem der vorhergehenden Ansprüche, **dadurch gekennzeichnet, dass** die wässrige Zusammensetzung ferner ein Tensid, insbesondere ein nichtionisches Tensid, mit der Maßgabe aufweist, dass der Entschäumer und das Tensid verschieden voneinander gewählt sind, wobei das nichtionische Tensid vorzugsweise ausgewählt ist aus der Gruppe bestehend aus Poloxamer, Fettalkoholalkoxylat wie Fettalkoholethoxylat, Polyvinylpyrrolidon, Alkylpolyglucosid und Kombinationen von wenigstens zwei der genannten nichtionischen Tenside.

11. Wässrige Zusammensetzung zur Anwendung nach einem der vorhergehenden Ansprüche, **dadurch gekennzeichnet, dass** das Tensid ein zwitterionisches Tensid, insbesondere ein Alkylamidoalkylbetain, bevorzugt ein Alkylamidoethylbetain, Alkylamidopropylbetain oder eine Kombination davon, ist.

12. Wässrige Zusammensetzung zur Anwendung nach einem der vorhergehenden Ansprüche, **dadurch gekennzeichnet, dass** die wässrige Zusammensetzung ferner einen Emulgator mit der Maßgabe aufweist, dass der Emulgator und ein optional vorhandenes Tensid voneinander verschieden gewählt sind, wobei das Tensid vorzugsweise ausgewählt ist aus der Gruppe bestehend aus Alkoholethoxylat, Alkylpolyglucosid, Polysorbat, ethoxyliertes Rizinusöl und Kombinationen von wenigstens zwei der genannten Emulgatoren.

13. Wässrige Zusammensetzung zur Anwendung nach einem der Ansprüche 10 bis 12, **dadurch gekennzeichnet, dass** das Tensid einen Aktiv-Anteil von 0.01 Gew.-% bis 10.0 Gew.-%, insbesondere 0.05 Gew.-% bis 2.0 Gew.-%, vorzugsweise 0.1 Gew.-% bis 1.0 Gew.-%, aufweist, bezogen auf das Gesamtgewicht der wässrigen Zusammensetzung, und/oder der Emulgator einen Anteil von 0.001 Gew.-% bis 1.5 Gew.-%, insbesondere 0.02 Gew.-% bis 1.0 Gew.-%, vorzugsweise 0.05 Gew.-% bis 0.5 Gew.-%, aufweist, bezogen auf das Gesamtgewicht der wässrigen Zusammensetzung.

14. Wässrige Zusammensetzung zur Anwendung nach einem der vorhergehenden Ansprüche, **dadurch gekennzeichnet, dass** die wässrige Zusammensetzung ferner einen Zusatzstoff, ausgewählt aus der Gruppe bestehend aus Verdickungsmittel, Komplexbildner, Feuchthaltemittel, Säure, Alkali, organische Lösungsmittel und Kombinationen von wenigstens zwei der genannten Zusatzstoffe, aufweist.

## Claims

1. An aqueous composition, especially in the form of an aqueous solution or a hydrogel, comprising
- a dihydrotriazine compound of the general formula I below: 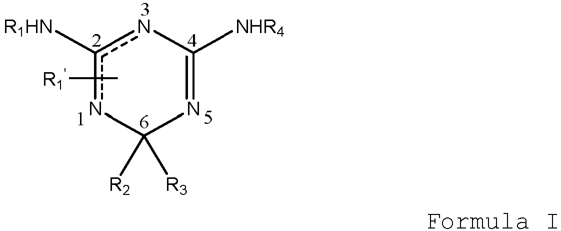 wherein
- R₁ means (i) a phenyl group or a phenylalkyl group, each of which is optionally substituted with 1 to 3 substituents selected from the group consisting of C₁₋₆-alkoxy group, hydroxy group, a halogen atom, C₁₋₆-haloalkyl group, C₁₋₆-alkyl group, a sulfonamido group and C₁₋₆-haloalkoxy group, (ii) a naphthyl group or a naphthylalkyl group, (iii) a heterocyclic group, a heterocyclic alkyl group or a heterocyclic aminoalkyl group, (iv) an alkyl group having 1 to 16 carbon atoms or (v) a cycloalkyl group or a cycloalkylalkyl group,
- R₁' means a hydrogen atom which is bonded to the nitrogen atom at position 1 or 3 of the dihydrotriazine ring,
- R₂ and R₃ independently of one another mean a hydrogen atom or a methyl group,
- R₄ means an alkyl group having 7 to 16 carbon atoms and
- the dashed line indicates that the position of a double bond is either between positions 1 and 2 or between positions 2 and 3 of the dihydrotriazine ring,
or a tautomer thereof or a salt thereof and
- a defoamer
for use in the prevention or treatment of infections and/or infectious diseases caused or partly caused by fungi.

2. The aqueous composition for use as claimed in claim 1, **characterized in that** R₁ means a phenyl group or a phenylalkyl group, especially benzyl group, each of which is optionally substituted by 1 to 3 substituents selected from the group consisting of fluorine atom, chlorine atom, hydroxy group, methyl group, tert-butyl group, trifluoromethyl group and methoxy group, R₁' means a hydrogen atom which is bonded to the nitrogen atom at position 1 or 3 of the dihydrotriazine ring, R₂ and R₃ both mean a methyl group and R₄ means an n-octyl group, n-nonyl group or n-decyl group.

3. The aqueous composition for use as claimed in claim 1 or 2, **characterized in that** R₁ means a phenyl group, 4-chlorophenyl group, 2,4-difluorophenyl group, 2,3,4-trifluorophenyl group, 4-tert-butylphenyl group, 4-methoxyphenyl group, 2-methoxy-4-tert-butylphenyl group, 4-trifluoromethoxyphenyl group, benzyl group, methylbenzyl group, 4-methylbenzyl group, 4-methoxybenzyl group, 3,4-dimethoxybenzyl group, 4-hydroxybenzyl group, 3,4-dichlorobenzyl group, 2,3,4-trichlorobenzyl group, 4-trifluoromethylbenzyl group, 1-phenylethyl group, 2-phenylethyl group, 1-phenylpropyl group, 2-phenylpropyl group or 3-phenylpropyl group, preferably a 4-methylbenzyl group, R₁' means a hydrogen atom which is bonded to the nitrogen atom at position 1 or 3 of the dihydrotriazine ring, R₂ and R₃ both mean a methyl group and R₄ means an n-octyl group, n-nonyl group or n-decyl group.

4. The aqueous composition for use as claimed in any of the preceding claims, **characterized in that** R₁ means a methylbenzyl group, preferably 4-methylbenzyl group, and/or R₂ and R₃ both mean a methyl group and/or R₄ means an n-octyl group.

5. The aqueous composition for use as claimed in any of the preceding claims, **characterized in that** R₁ means a methylbenzyl group, preferably 4-methylbenzyl group, R₁' means a hydrogen atom which is bonded to the nitrogen atom at position 1 or 3 of the dihydrotriazine ring, R₂ and R₃ both mean a methyl group and R₄ means an n-octyl group.

6. The aqueous composition for use as claimed in any of the preceding claims, **characterized in that** the dihydrotriazine compound is 4-octylamino-1,6-dihydro-6,6-dimethyl-2-(4'-methylbenzylamino)-1,3,5-triazine gluconate, 4-octylamino-3,6-dihydro-6,6-dimethyl-2-(4'-methylbenzylamino)-1,3,5-triazine gluconate, or a tautomer thereof.

7. The aqueous composition for use as claimed in any of the preceding claims, **characterized in that** the dihydrotriazine compound has a proportion of of 0.001% by weight to 1.00% by weight, especially 0.01% by weight to 0.50% by weight, preferably 0.025% by weight to 0.25% by weight, based on the total weight of the aqueous composition.

8. The aqueous composition for use as claimed in any of the preceding claims, **characterized in that** the defoamer is selected from the group consisting of alkyl amide, silicone, poloxamer and combinations of at least two of the defoamers mentioned.

9. The aqueous composition for use as claimed in any of the preceding claims, **characterized in that** the defoamer has a proportion of 0.0001% by weight to 2.0% by weight, especially 0.0005% by weight to 1.5% by weight, preferably 0.001% by weight to 1.0% by weight, based on the total weight of the aqueous composition.

10. The aqueous composition for use as claimed in any of the preceding claims, **characterized in that** the aqueous composition further comprises a surfactant, especially a nonionic surfactant, with the proviso that the defoamer and the surfactant are chosen such that they are different from one another, wherein the nonionic surfactant is preferably selected from the group consisting of poloxamer, fatty alcohol alkoxylate such as fatty alcohol ethoxylate, polyvinylpyrrolidone, alkyl polyglucoside and combinations of at least two of the nonionic surfactants mentioned.

11. The aqueous composition for use as claimed in any of the preceding claims, **characterized in that** the surfactant is a zwitterionic surfactant, especially an alkylamidoalkyl betaine, preferably an alkylamidoethyl betaine, alkylamidopropyl betaine or a combinations thereof.

12. The aqueous composition for use as claimed in any of the preceding claims, **characterized in that** the aqueous composition further comprises an emulsifier, with the proviso that the emulsifier and an optionally present surfactant are chosen such that they are different from one another, wherein the surfactant is preferably selected from the group consisting of alcohol ethoxylate, alkyl polyglucoside, polysorbate, ethoxylated castor oil and combinations of at least two of the emulsifiers mentioned.

13. The aqueous composition for use as claimed in any of claims 10 to 12, **characterized in that** the surfactant has an active proportion of 0.01% by weight to 10.0% by weight, especially 0.05% by weight to 2.0% by weight, preferably 0.1% by weight to 1.0% by weight, based on the total weight of the aqueous composition, and/or the emulsifier has a proportion of 0.001% by weight to 1.5% by weight, especially 0.02% by weight to 1.0% by weight, preferably 0.05% by weight to 0.5% by weight, based on the total weight of the aqueous composition.

14. The aqueous composition for use as claimed in any of the preceding claims, **characterized in that** the aqueous composition further comprises an additive selected from the group consisting of thickener, complexing agent, humectant, acid, alkali, organic solvent and combinations of at least two of the additives mentioned.

## Revendications

1. Composition aqueuse, en particulier sous forme d'une solution aqueuse ou d'un hydrogel, présentant
- un composé dihydrotriazine de la formule générale I suivante : 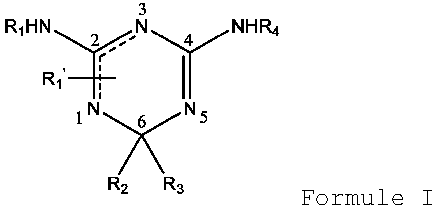 dans laquelle
- R₁ signifie (i) un groupe phényle ou un groupe phénylalkyle dont chacun est éventuellement substitué par 1 à 3 substituants, choisis dans le groupe constitué par un groupe C₁₋₆-alcoxy, un groupe hydroxy, un atome d'halogène, un groupe C₁₋₆-halogénoalkyle, un groupe C₁₋₆-alkyle, un groupe sulfonamido et un groupe C₁₋₆-halogénoalcoxy, (ii) un groupe naphtyle ou un groupe naphtylalkyle, (iii) un groupe hétérocyclique, un groupe alkyle hétérocyclique ou un groupe aminoalkyle hétérocyclique, (iv) un groupe alkyle comprenant 1 à 16 atomes de carbone ou (v) un groupe cycloalkyle ou un groupe cycloalkyl-alkyle,
- R₁' signifie un atome d'hydrogène, qui est lié en position 1 ou 3 du cycle dihydrotriazine à l'atome d'azote,
- R₂ et R₃ signifient, indépendamment l'un de l'autre, un atome d'hydrogène ou un groupe méthyle,
- R₄ signifie un groupe alkyle comprenant 7 à 16 atomes de carbone et
- la ligne en pointillés indique que la position d'une double liaison se situe soit entre les positions 1 et 2, soit entre les positions 2 et 3 du cycle dihydrotriazine, ou un tautomère ou un sel de celui-ci et
- un antimousse
pour une utilisation lors de la prévention ou du traitement d'infections et/ou de maladies infectieuses provoquées par des champignons ou dans lesquelles ceux-ci sont impliqués.

2. Composition aqueuse pour une utilisation selon la revendication 1, **caractérisée en ce que** R₁ signifie un groupe phényle ou un groupe phénylalkyle, en particulier un groupe benzyle, dont chacun est éventuellement substitué par 1 à 3 substituants choisis dans le groupe constitué par un atome de fluor, un atome de chlore, un groupe hydroxy, un groupe méthyle, un groupe tert-butyle, un groupe trifluorométhyle et un groupe méthoxy, R₁' signifie un atome hydrogène, qui est lié en position 1 ou 3 du cycle dihydrotriazine à l'atome d'azote, R₂ et R₃ signifient à chaque fois un groupe méthyle et R₄ signifie un groupe n-octyle, un groupe n-nonyle ou un groupe n-décyle.

3. Composition aqueuse pour une utilisation selon la revendication 1 ou 2, **caractérisée en ce que** R₁ signifie un groupe phényle, un groupe 4-chlorophényle, un groupe 2,4-difluorophényle, un groupe 2,3,4-trifluorophényle, un groupe 4-tert-butylphényle, un groupe 4-méthoxyphényle, un groupe 2-méthoxy-4-tert-butylphényle, un groupe 4-trifluorométhoxyphényle, un groupe benzyle, un groupe méthylbenzyle, un groupe 4-méthylbenzyle, un groupe 4-méthoxybenzyle, un groupe 3,4-diméthoxybenzyle, un groupe 4-hydroxybenzyle, un groupe 3,4-dichlorobenzyle, un groupe 2,3,4-trichlorobenzyle, un groupe 4-trifluorométhylbenzyle, un groupe 1-phényléthyle, un groupe 2-phényléthyle, un groupe 1-phénylpropyle, un groupe 2-phénylpropyle ou un groupe 3-phénylpropyle, de préférence un groupe 4-méthylbenzyle, R₁' signifie un atome hydrogène, qui est lié en position 1 ou 3 du cycle dihydrotriazine à l'atome d'azote, R₂ et R₃ signifient à chaque fois un groupe méthyle et R₄ signifie un groupe n-octyle, un groupe n-nonyle ou un groupe n-décyle.

4. Composition aqueuse pour une utilisation selon l'une des revendications précédentes, **caractérisée en ce que** R₁ signifie un groupe méthylbenzyle, de préférence un groupe 4-méthylbenzyle, et/ou R₂ et R₃ signifient à chaque fois un groupe méthyle et/ou R₄ signifie un groupe n-octyle.

5. Composition aqueuse pour une utilisation selon l'une des revendications précédentes, **caractérisée en ce que** R₁ signifie un groupe méthylbenzyle, de préférence un groupe 4-méthylbenzyle, R₁' signifie un atome hydrogène, qui est lié en position 1 ou 3 du cycle dihydrotriazine à l'atome d'azote, R₂ et R₃ signifient à chaque fois un groupe méthyle et R₄ signifie un groupe n-octyle.

6. Composition aqueuse pour une utilisation selon l'une des revendications précédentes, **caractérisée en ce qu'**il s'agit, pour le composé dihydrotriazine, de gluconate de 4-octylamino-1,6-dihydro-6,6-diméthyl-2-(4'-méthylbenzylamino)-1,3,5-triazine, de gluconate de 4-octylamino-3,6-dihydro-6,6-diméthyl-2-(4'-méthylbenzylamino)-1,3,5-triazine ou d'un tautomère de ceux-ci.

7. Composition aqueuse pour une utilisation selon l'une des revendications précédentes, **caractérisée en ce que** le composé dihydrotriazine représente une proportion de 0,001% en poids à 1,00% en poids, en particulier de 0,01% en poids à 0,50% en poids, de préférence de 0,025% en poids à 0,25% en poids, par rapport au poids total de la composition aqueuse.

8. Composition aqueuse pour une utilisation selon l'une des revendications précédentes, **caractérisée en ce que** l'antimousse est choisi dans le groupe constitué par un alkylamide, une silicone, un poloxamère et les combinaisons d'au moins deux des antimousses mentionnés.

9. Composition aqueuse pour une utilisation selon l'une des revendications précédentes, **caractérisée en ce que** l'antimousse représente une proportion de 0,0001% en poids à 2,0% en poids, en particulier de 0,0005% en poids à 1,5% en poids, de préférence de 0,001% en poids à 1,0% en poids, par rapport au poids total de la composition aqueuse.

10. Composition aqueuse pour une utilisation selon l'une des revendications précédentes, **caractérisée en ce que** la composition aqueuse présente en outre un tensioactif, en particulier un tensioactif non ionique, étant entendu que l'antimousse et le tensioactif sont choisis différemment l'un de l'autre, le tensioactif non ionique étant de préférence choisi dans le groupe constitué par un poloxamère, un alcoxylate d'alcool gras tel qu'un éthoxylate d'alcool gras, la polyvinylpyrrolidone, un alkylpolyglucoside et les combinaisons d'au moins deux des tensioactifs non ioniques mentionnés.

11. Composition aqueuse pour une utilisation selon l'une des revendications précédentes, **caractérisée en ce que** le tensioactif est un tensioactif zwittérionique, en particulier une alkylamidoalkylbétaïne, de préférence une alkylamidoéthylbétaïne, une alkylamidopropylbétaïne ou une combinaison de celles-ci.

12. Composition aqueuse pour une utilisation selon l'une des revendications précédentes, **caractérisée en ce que** la composition aqueuse présente en outre un émulsifiant, étant entendu que l'émulsifiant et un tensioactif éventuellement présent sont choisis différemment l'un de l'autre, le tensioactif étant de préférence choisi dans le groupe constitué par un éthoxylate d'alcool, un alkylpolyglucoside, un polysorbate, l'huile de ricin éthoxylée et les combinaisons d'au moins deux des émulsifiants mentionnés.

13. Composition aqueuse pour une utilisation selon l'une des revendications 10 à 12, **caractérisée en ce que** le tensioactif présente une proportion active de 0,01% en poids à 10,0% en poids, en particulier de 0,05% en poids à 2,0% en poids, de préférence de 0,1% en poids à 1,0% en poids, par rapport au poids total de la composition aqueuse et/ou l'émulsifiant représente une proportion de 0,001% en poids à 1,5% en poids, en particulier de 0,02% en poids à 1,0% en poids, de préférence de 0,05% en poids à 0,5% en poids, par rapport au poids total de la composition aqueuse.

14. Composition aqueuse pour une utilisation selon l'une des revendications précédentes, **caractérisée en ce que** la composition aqueuse présente en outre un additif choisi dans le groupe constitué par les épaississants, les complexants, les agents de rétention d'eau, les acides, les alcalis, les solvants organiques et les combinaisons d'au moins deux des additifs mentionnés.
